# EUROPEAN PATENT APPLICATION

(11) **EP 1 721 609 A2**
(43) Date of publication of application: **15.11.2006**
(21) Application number: 06013237.0
(22) Date of filing: 16.05.2002
(51) Int. Cl.: A61K 31/506, G01N 33/68, C12Q 1/42, C12Q 1/48, C07K 16/40, C12N 15/11, A61P 25/28, C07D 401/04, C07D 409/14, C07D 401/14, C07D 471/04

(54) **Pyridylpyrimidine derivatives as effective compounds against prion infections and prion diseases**

(30) Priority: 16.05.2001 EP 01111858; 29.05.2001 US 293528 P; 13.07.2001 EP 01117113; 18.07.2001 US 305898 P
(62) Divisional of application: 02769490.0
(71) Applicant: GPC Biotech AG, 82152 Martinsried (DE)
(72) Inventor: Stein-Gerlach, Matthias, 81475 München (DE); Salassidis, Konstadinos, 85386 Eching (DE); Bacher, Gerald, 82110 Germering (DE); Müller, Stefan, 81371 München (DE); Klebl, Bert, 82294 Günzelhofen (DE); Marosfalvi, Jenö, 1118 Budapest (HU); Keri, György, 1021 Budapest (HU); Örfi, Laszlo, 1161 Budapest (HU); Varga, Zoltan, 7052 Kölesd (HU)
(74) Representative: Dörries, Hans Ulrich

(57) **Abstract**

The present invention relates to pyridylpyrimidine derivatives of the general formula (I): 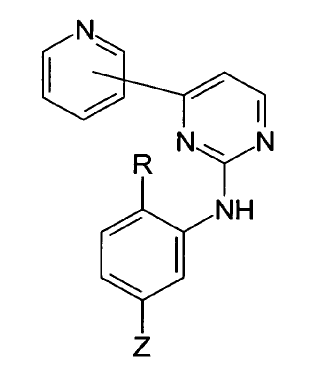 wherein R represents hydrogen or methyl and Z represents nitrogen containing functional groups, the use of the pyridylpyrimidine derivatives as pharmaceutically active agents, especially for the prophylaxis and/or treatment of prion infections and prion diseases, as well as compositions containing at least one pyridylpyrimidine derivative and/or pharmaceutically acceptable salt thereof. Furthermore, the present invention is directed to methods for preventing and/or treating prion infections and prion diseases using said pyridylpyrimidine derivatives. Human cellular protein kinases, phosphatases and cellular signal transduction molecules are disclosed as targets for detecting, preventing and/or treating prion infections and diseases, especially BSE, vCJD, or CJD which can be inhibited by the inventive pyridylpyrimidine derivatives.

## Description

The present invention relates to pyridylpyrimidine derivatives, the use of the pyridylpyrimidine derivatives as pharmaceutically active agents, especially for the prophylaxis and/or treatment of prion infections and prion diseases, as well as compositions containing at least one pyridylpyrimidine derivative and/or pharmaceutically acceptable salt thereof, and methods for preventing and/or treating prion infections and prion diseases. Furthermore, human cellular protein kinases, phosphatases and cellular signal transduction molecules are disclosed as targets for detecting, preventing and/or treating prion infections and diseases, especially BSE, vCJD, or CJD.

### Background of the invention

Pyridylpyrimidine derivatives are known from WO 9509851 as effective compounds for chemotherapy of tumors, from WO 9509853, EP-A-0 588 762, WO 9509847, WO 9903854, and EP-B-0 564 409 as effective compounds for treatment of tumors. Furthermore, EP-B-0 564 409 discloses the use of said compounds in the treatment of artherosclerosis and Exp. Opin. Ther. Patents, 1998, 8(12), 1599-1625 describes the use of pyridylpyrimidine derivatives, especially of Gleevec™, the Novartis compound CGP 57148, as tyrosine kinase inhibitors in cancer treatment.

Prions are infectious agents which do not have a nucleic acid genome. It seems that a protein alone is the infectious agent. A prion has been defined as "small proteinaceous infectious particle which resists inactivation by procedures that modify nucleic acids". The discovery that proteins alone can transmit an infectious disease has come as a considerable surprise to the scientific community. Prion diseases are often called "transmissible spongiform encephalopathies", because of the post mortem appearance of the brain with large vacuoles in the cortex and cerebellum. Probably most mammalian species develop these diseases. Prion diseases are a group of neurodegenerative disorders of humans and animals and the prion diseases can manifest as sporadic, genetic or infectious disorders. Examples for prion diseases acquired by exogenous infection are the Bovine spongiform encephalitis (BSE) of cattle and the new variant of Creutzfeld-Jakob disease (vCJD) caused by BSE. Further examples include kuru, Gerstmann-Sträussler-Scheinker disease of humans as well as scrapie of animals. For many years, the prion diseases were thought to be caused by viruses despite intriguing evidence to the contrary. The unique characteristic common to all of these disorders, whether sporadic, dominantly inherited, or acquired by infection, is that they involve the aberrant metabolism of the prion protein (PrP). In many cases, the cellular prion protein (PrP^{c}) ["c" refers to cellular] is converted into the scrapie isoform (PrP^{Sc}) ["Sc" refers to Scrapie] by a posttranslational process that involves a conformational change. Often, the human prion diseases are transmissible to experimental animals and all of the inherited prion diseases segregate with PrP gene mutations.

These prion diseases in animals and humans have a long incubation period and a long clinical course, and are always fatal leading via decerebration to death within an average period of 7 months (CJD). Neuropathological features consist of neuronal vacuolization, neuronal death and gliosis with hyperastrocytosis. The precise diagnosis of transmissible neurodegenerative diseases can be established only by the examination of the central nervous system after biopsy or autopsy.

Clinical symptoms of the disease are progressive dementia, myoclonus and prominent ataxia with the additional clinical features of dysautonomia and delirious psychomotor excitement and with relatively preserved verbal responses.

Between 1980 and, roughly, 1996, about 750,000 cattle infected with BSE were slaughtered for human consumption in Great Britain (Anderson, R. M. et al. Nature 382, 779-788,1996; Ferguson, N. M., Donneiiy, C. A., Woolhouse, M. E. J. & Anderson, R. M. Phil. Trans. R. Soc. Lond. B 352, 803-838, 1997). The annual incidence of vCJD (3, 10, 10, 18, 14 and 33 deaths in 1995-2000, respectively) can be interpreted as a first sign of a steady or exponential increase over the next years. The suggestion by the European Union Scientific Steering Committee that up to 500,000 people could have been exposed to BSE from a single infected bovine has fuelled speculation that millions of consumers are at risk.

Recent findings demonstrate that the pathogenic PrP^{Sc} of vCJD can be found in the lymph system (e.g. tonsils, lymph nodes) in humans suggesting a high risk of horizontal spread via lymph and/or blood transmission, dramatically increasing the number of people at risk.

The medical need in prion diseases today can be clearly defined as the establishment of a diagnostic system, that can detect the disease as early as possible in living humans and/or animals, to estimate the medical need for the treatment in the future and to identify the infected animals to remove them from the food chain. The medical need for prion diseases in the future (approximately starting in 5-10 years) will be medical treatment that inhibits the disease symptoms, the manifestation and/or progression of the disease.

It is object of the present invention to provide novel and also known compounds which can be used as pharmaceutically active agents, especially for prophylaxis and/or treatment of prion infections and prion diseases, methods wherein said compounds are used in order to treat prion infections and prion diseases and compositions containing at least one inventive compound and/or pharmaceutically acceptable salt thereof as a pharmaceutically active ingredient.

The object of the present invention is solved by the teaching of the independent claims. Further advantageous features, aspects and details of the invention are evident from the dependent claims, the description, the examples, and the figures of the present application.

### Description of the invention

One aspect of the present invention is related to compounds of the general formula (I): 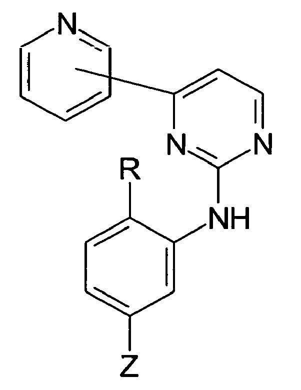 wherein:
R represents hydrogen or methyl;
Y, Y', Y" are independently of each other ―H, ―F, ―Cl, ―Br, ―I, ―CH₂F, ―CH₂Cl, ―CH₂Br, ―CH₂I, ―OH, ―OCH₃, ―CH₃, ―CN, ―OCF₃, 4-methylpiperazin-1-yl-methyl, ―C(CH₃)=N―NH―C(NH)NH₂;
Z represents ―NO₂, ―NH₂, -NH―CO―X, ―NH―CS―X, ―NH―CO―NH―X, ―NH―SO₂―X;
X represents thiophenyl, cyclohexyl, isoquinolinyl, naphthyl, quinolinyl, cyclopentyl, pyridinyl, naphthyridinyl, or 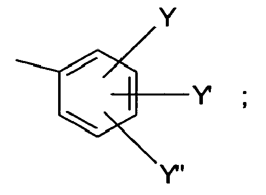 and pharmaceutically acceptable salts thereof.

Another aspect of the present invention relates to the use of compounds of the general formula (I): 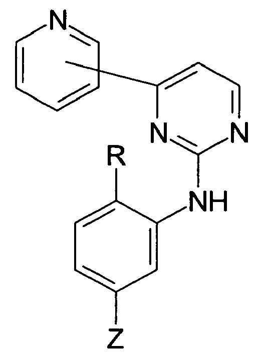 wherein:
R represents hydrogen or methyl;
Y, Y', Y" are independently of each other -H, -F, -Cl, -Br, -I, -CH₂F, -CH₂Cl, -CH₂Br, -CH₂l, -OH, -OCH₃, -CH₃, -CN, -OCF₃, 4-methylpiperazin-1-yl-methyl, -C(CH₃)=N-NH-C(NH)-NH₂;
Z represents -NO₂, -NH₂, -NH-CO-X, -NH-CS-X, -NH-CO-NH-X, -NH-SO₂-X;
X represents thiophenyl, cyclohexyl, isoquinolinyl, naphthyl, quinolinyl, cyclopentyl, pyridinyl, naphthyridinyl, or 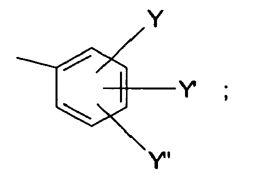 and pharmaceutically acceptable salts thereof as pharmaceutically active agents, especially for prophylaxis and/or treatment of infectious diseases, or in a more general sense, for prophylaxis and/or treatment of nerodegenerative diseases.

Thus, one embodiment of the present invention disclosed herein is directed to a method for preventing and/or treating infections and/or diseases associated with said infections in an individual. Said method comprises administering to the individual an amount of at least one compound according to general formula (I) and/or pharmaceutically acceptable salts thereof effective to prevent and/or treat said infections and/or diseases. Most preferred is the administration of a compound 53.

As revealed for the first time herein, the present invention discloses the use of compounds of the general formula (I) for the prophylaxis and/or treatment of prion infections and prion diseases. As described above, said pyridylpyrimidine derivatives have first of all been used in tumor therapy. The Novartis compound Gleevec™ also known as Glivec™, CGP-57148B, imatinib mesylate, STI-571, STI-571A, CAS 152459-95-5, or 4-((Methyl-1-piperazinyl)methyl)-N-[4-methyl-3-[[4-(3-pyridinyl)-2-pyrimidinyl]amino]-phenyl]benzamide methanesulfonate, has been registered in many countries as anticancer drug. This Gleevec™ compound (compound 53) is also the most active one in the indication prion diseases.

The name "prion" is used to describe the causative agents which underlie the transmissible spongiform encephalopathies. A prion is proposed to be a novel infectious particle that differs from viruses and viroids. It is composed solely of one unique protein that resists most inactivation procedures such as heat, radiation, and proteases. The latter characteristic has led to the term protease-resistant isoform of the prion protein. The protease-resistant isoform has been proposed to slowly catalyze the conversion of the normal prion protein into the abnormal form.

The term "isoform" in the context of prions means two proteins with exactly the same amino acid sequence that are folded into molecules with dramatically different tertiary structures. The normal cellular isoform of the prion protein (PrP^{c}) has a high α-helix content, a low β-sheet content, and is sensitive to protease digestion. The abnormal, disease-causing isoform (PrP^{Sc}) has a lower α-helix content, a much higher β-sheet content, and is much more resistant to protease digestion.

Moreover, in a more general sense, the present invention is concerned with the prophylaxis ans/or treatment of neurodegenerative diseases. For example, Alzheimer is a well-known neurodegenerative disease.

Preferred are the compounds wherein R represents hydrogen. Also preferred are compounds wherein Z represents ―NH―CO―X or ―NH―SO₂―X and/or wherein Y, Y', Y" are independently of each other ―H, ―F, ―Cl, ―CH₂F, ―CH₂Cl, ―OH, ―OCH₃, ―CN, ―OCF₃, or a 4-methylpiperazin-1-yl-methyl residue.

Also preferred are the following pyridylpyrimidine derivatives selected from the group comprising:
- Compound 1:: (3-Nitrophenyl)-(4-pyridin-3-yl-pyrimidin-2-yl)-amine;
- Compound 2:: (3-Aminophenyl)-(4-pyridin-3-yl-pyrimidin-2-yl)-amine;
- Compound 3:: (5-Amino-2-methylphenyl)-(4-pyridin-3-yl-pyrimidin-2-yl)-amine;
- Compound 4:: 4-Chloromethyl-*N*-[4-methyl-3-(4-pyridin-3-yl-pyrimidin-2-ylamino)-phenyl]-benzamide;
- Compound 5:: 4-Chloromethyl-*N*-[3-(4-pyridin-3-yl-pyrimidin-2-ylamino)-phenyl]-benzamide;
- Compound 6:: 4-(4-Methylpiperazin-1-ylmethyl)-*N*-[3-(4-pyridin-3-yl-pyrimidin-2-ylamino)-phenyl]-benzamide;
- Compound 7:: Thiophene-3-carboxylic acid [4-methyl-3-(4-pyridin-4-yl-pyrimidin-2-ylamino)-phenyl]-amide;
- Compound 8:: 4-Chloro-*N*-[4-methyl-3-(4-pyridin-3-yl-pyrimidin-2-ylamino)-phenyl]-benzamide;
- Compound 9:: 4-Chloro-*N*-[3-(4-pyridin-3-yl-pyrimidin-2-ylamino)-phenyl]-benzamide;
- Compound 10:: 3,4,5-Trimethoxy-*N*-[3-(4-pyridin-3-yl-pyrimidin-2-ylamino)-phenyl]-benzamide;
- Compound 11:: 4-Cyano-*N*-[3-(4-pyridin-3-yl-pyrimidin-2-ylamino)-phenyl]-benzamide;
- Compound 12:: 4-Methoxy-*N*-[3-(4-pyridin-3-yl-pyrimidin-2-ylamino)-phenyl]-benzamide;
- Compound 13:: 4-Chloro-*N*-[3-(4-pyridin-3-yl-pyrimidin-2-ylamino)-phenyl]-benzenesulfonamide;
- Compound 14:: Thiophene-3-carboxylic acid [3-(4-pyridin-3-yl-pyrimidin-2-ylamino)-phenyl]-amide;
- Compound 15:: 3,5-Dimethoxy-*N*-[3-(4-pyridin-3-yl-pyrimidin-2-ylamino)-phenyl]-benzamide;
- Compound 16:: 3,4,5-Trimethoxy-*N*-[4-methyl-3-(4-pyridin-3-yl-pyrimidin-2-ylamino)-phenyl]-benzamide;
- Compound 17:: 4-Cyano-*N*-[4-methyl-3-(4-pyridin-3-yl-pyrimidin-2-ylamino)-phenyl]-benzamide;
- Compound 18:: 4-Methoxy-*N*-[4-methyl-3-(4-pyridin-3-yl-pyrimidin-2-ylamino)-phenyl]-benzamide;
- Compound 19:: 4-Chloro-*N*-[4-methyl-3-(4-pyridin-3-yl-pyrimidin-2-ylamino)-phenyl]-benzenesulfonamide;
- Compound 20:: Thiophene-3-carboxylic acid [4-methyl-3-(4-pyridin-3-yl-pyrimidin-2-ylamino)-phenyl]-amide;
- Compound 21:: 3,5-Dimethoxy-*N*-[4-methyl-3-(4-pyridin-3-yl-pyrimidin-2-ylamino)-phenyl]-benzamide;
- Compound 22:: 4-Trifluoromethoxy-*N*-[4-methyl-3-(4-pyridin-3-yl-pyrimidin-2-ylamino)-phenyl]-benzamide;
- Compound 23:: Cyclohexanecarboxylic acid [4-methyl-3-(4-pyridin-3-yl-pyrimidin-2-ylamino)-phenyl]-amide;
- Compound 24:: Cyclohexanecarboxylic acid [3-(4-pyridin-3-yl-pyrimidin-2-ylamino)-phenyl]-amide;
- Compound 25:: Isoquinoline-5-sulfonic acid [4-methyl-3-(4-pyridin-3-yl-pyrimidin-2-ylamino)-phenyl]-amide;
- Compound 26:: Isoquinoline-5-sulfonic acid [3-(4-pyridin-3-yl-pyrimidin-2-ylamino)-phenyl]-amide;
- Compound 27:: (5-Nitro-2-methylphenyl)-(4-pyridin-2-yl-pyrimidin-2-yl)-amine;
- Compound 28:: (5-Amino-2-methylphenyl)-(4-pyridin-2-yl-pyrimidin-2-yl)-amine;
- Compound 29:: 3,4,5-Trimethoxy-*N*-[4-methyl-3-(4-pyridin-2-yl-pyrimidin-2-ylamino)-phenyl]-benzamide;
- Compound 30:: 4-Cyano-*N*-[4-methyl-3-(4-pyridin-2-yl-pyrimidin-2-ylamino)-phenyl]-benzamide;
- Compound 31:: (3-Aminophenyl)-(4-pyridin-2-yl-pyrimidin-2-yl)-amine;
- Compound 32:: 4-Chloro-*N*-[4-methyl-3-(4-pyridin-2-yl-pyrimidin-2-ylamino)-phenyl]-benzamide;
- Compound 33:: Cyclohexanecarboxylic acid [4-methyl-3-(4-pyridin-2-yl-pyrimidin-2-ylamino)-phenyl]-amide;
- Compound 34:: 4-Cyano-*N*-[4-methyl-3-(4-pyridin-4-yl-pyrimidin-2-ylamino)-phenyl]-benzamide;
- Compound 35:: 4-Chloro-*N*-[4-methyl-3-(4-pyridin-4-yl-pyrimidin-2-ylamino)-phenyl]-benzenesulfonamide;
- Compound 36:: 4-Methoxy-*N*-[4-methyl-3-(4-pyridin-4-yl-pyrimidin-2-ylamino)-phenyl]-benzamide;
- Compound 37:: 4-Chloro-*N*-[4-methyl-3-(4-pyridin-4-yl-pyrimidin-2-ylamino)-phenyl]-benzamide;
- Compound 38:: Cyclohexanecarboxylic acid [3-(4-pyridin-4-yl-pyrimidin-2-ylamino)-phenyl]-amide;
- Compound 39:: 3,5-Dimethoxy-*N*-[3-(4-pyridin-4-yl-pyrimidin-2-ylamino)-phenyl]-benzamide;
- Compound 40:: (5-Amino-2-methylphenyl)-(4-pyridin-4-yl-pyrimidin-2-yl)-amine;
- Compound 41:: Thiophene-3-carboxylic acid [3-(4-pyridin-4-yl-pyrimidin-2-ylamino)-phenyl]-amide;
- Compound 42:: 4-Chloro-*N*-[3-(4-pyridin-4-yl-pyrimidin-2-ylamino)-phenyl]-benzenesulfonamide;
- Compound 43:: 4-Chloro-*N*-[3-(4-pyridin-4-yl-pyrimidin-2-ylamino)-phenyl]-benzamide;
- Compound 44:: (3-Aminophenyl)-(4-pyridin-4-yl-pyrimidin-2-yl)-amine;
- Compound 45:: (3-Nitrophenyl)-(4-pyridin-4-yl-pyrimidin-2-yl)-amine;
- Compound 46:: 4-Trifluoromethoxy-*N*-[4-methyl-3-(4-pyridin-4-yl-pyrimidin-2-ylamino)-phenyl]-benzamide;
- Compound 47:: Isoquinoline-5-sulfonic acid [3-(4-pyridin-4-yl-pyrimidin-2-ylamino)-phenyl]-amide;
- Compound 48:: 4-Methoxy-*N*-[3-(4-pyridin-4-yl-pyrimidin-2-ylamino)-phenyl]-benzamide;
- Compound 49:: 4-Cyano-*N*-[3-(4-pyridin-4-yl-pyrimidin-2-ylamino)-phenyl]-benzamide;
- Compound 50:: 3,4,5-Trimethoxy-*N*-[3-(4-pyridin-4-yl-pyrimidin-2-ylamino)-phenyl]-benzamide;
- Compound 51:: 3,5-Dimethoxy-*N*-[4-methyl-3-(4-pyridin-4-yl-pyrimidin-2-ylamino)-phenyl]-benzamide;
- Compound 52:: 3,4,5-Trimethoxy-*N*-[4-methyl-3-(4-pyridin-4-yl-pyrimidin-2-ylamino)-phenyl]-benzamide;
- Compound 53:: 4-(4-Methylpiperazin-1-ylmethyl)-*N*-[4-methyl-3-(4-pyridin-3-yl-pyrimidin-2-ylamino)-phenyl]-benzamide (Gleevec™);
- Compound 54:: 4-Methyl-*N*-[4-methyl-3-(4-pyridin-2-yl-pyrimidin-2-ylamino)-phenyl]-benzenesulfonamide
- Compound 55:: 4-Methoxy-*N*-[4-methyl-3-(4-pyridin-2-yl-pyrimidin-2-ylamino)-phenyl]-benzamide;
- Compound 56:: 3,5-Dimethoxy-*N*-[4-methyl-3-(4-pyridin-2-yl-pyrimidin-2-ylamino)-phenyl]-benzamide;
- Compound 57:: Naphthalene-2-carboxylic acid [4-methyl-3-(4-pyridin-3-yl-pyrimidin-2-ylamino)-phenyl]-amide;
- Compound 58:: *N*-[3-(4-Pyridin-4-yl-pyrimidin-2-ylamino)-phenyl]-benzamide;
- Compound 59:: 4-Chloro-*N*-[3-(4-pyridin-2-yl-pyrimidin-2-ylamino)-phenyl]-benzamide;
- Compound 60:: 4-Methoxy-*N*-[3-(4-pyridin-2-yl-pyrimidin-2-ylamino)-phenyl]-benzamide;
- Compound 61:: 4-Chloro-*N*-[3-(4-pyridin-2-yl-pyrimidin-2-ylamino)-phenyl]-benzenesulfonamide;
- Compound 62:: Thiophene-2-carboxylic acid 3-(4-pyridin-2-yl-pyrimidin-2-yl-amino)-phenyl]-amide;
- Compound 63:: Naphthalene-2-sulfonic-acid [3-(4-pyridin-2-yl-pyrimidin-2-yl-amino)-phenyl]-amide;
- Compound 64:: Isoquinoline-5-sulfonic-acid [3-(4-pyridin-2-yl-pyrimidin-2-yl-amino)-phenyl]-amide;
- Compound 65:: Cylopentanecarboxylic acid 3-(4-pyridin-2-yl-pyrimidin-2-yl-amino)-phenyl]-amide;
- Compound 66:: Naphthalene-2-carboxylic acid [3-(4-pyridin-2-yl-pyrimidin-2-ylamino)-phenyl]-amide;
- Compound 67:: 4-Cyano-*N*-[3-(4-pyridin-2-yl-pyrimidin-2-ylamino)-phenyl]-benzamide;
- Compound 68:: 3,5-Dimethoxy-*N*-[3-(4-pyridin-2-yl-pyrimidin-2-ylamino)-phenyl]-benzamide;
- Compound 69:: 4-Bromo-*N*-[3-(4-pyridin-2-yl-pyrimidin-2-ylamino)-phenyl]-benzamide;
- Compound 70:: 4-Methyl-*N*-[3-(4-pyridin-2-yl-pyrimidin-2-ylamino)-phenyl]-benzamide;
- Compound 71:: 4-Fluoro-*N*-3-(4-pyridin-2-yl-pyrimidin-2-ylamino)-phenyl]-benzenesulfonamide;
- Compound 72:: 3,5-Dichloro-*N*-[3-(4-pyridin-2-yl-pyrimidin-2-ylamino)-phenyl]-benzamide;
- Compound 73:: *N*-[3-(4-Pyridin-2-yl-pyrimidin-2-ylamino)-phenyl]-benzamide;
- Compound 74:: 4-Chloromethyl-*N*-[3-(4-pyridin-2-yl-pyrimidin-2-ylamino)-phenyl]-benzamide;
- Compound 75:: 4-Methyl-*N*-3-(4-pyridin-2-yl-pyrimidin-2-ylamino)-phenyl]-benzenesulfonamide
- Compound 76:: 4-(4-Methylpiperazin-1-ylmethyl)-*N*-[3-(4-pyridin-2-yl-pyrimidin-2-ylamino)-phenyl]-benzamide;
- Compound 77:: Naphthalene-2-carboxylic acid [3-(4-pyridin-4-yl-pyrimidin-2-ylamino)-phenyl]-amide;
- Compound 78:: 2-Methoxy-*N*-[4-methyl-3-(4-pyridin-3-yl-pyrimidin-2-ylamino)-phenyl]-benzamide;
- Compound 79:: 2-Methoxy-*N*-[3-(4-pyridin-3-yl-pyrimidin-2-ylamino)-phenyl]-benzamide;
- Compound 80:: 4-Methyl-*N*-[3-(4-pyridin-3-yl-pyrimidin-2-ylamino)-phenyl]-benzamide;
- Compound 81:: 4-Methyl-*N*-[4-methyl-3-(4-pyridin-3-yl-pyrimidin-2-ylamino)-phenyl]-benzamide;
- Compound 82:: *N*-[4-Methyl-3-(4-pyridin-3-yl-pyrimidin-2-ylamino)-phenyl]-benzamide;
- Compound 83:: 1-(3,5-Diacetyl-phenyl)-3-[4-methyl-3-(4-pyridin-3-yl-pyrimidin-2-ylamino)-phenyl]-urea;
- Compound 84:: 1-{3,5-Bis-(amidinohydrazone)-phenyl}-3-[4-methyl-3-(4-pyridin-3-yl-pyrimidin-2-ylamino)-phenyl]-urea;
- Compound 85:: *N*-[4-Methyl-3-(4-pyridin-3-yl-pyrimidin-2-ylamino)-phenyl]-nicotinamide;
- Compound 86:: *N*-[3-(4-Pyridin-3-yl-pyrimidin-2-ylamino)-phenyl]-nicotinamide;
- Compound 87:: [1,8]Naphthyridine-2-carboxylic acid [3-(4-pyridin-3-yl-pyrimidin -2-ylamino)-phenyl]-amide;
- Compound 88:: [1,8]Naphthyridine-2-carbothioic acid [3-(4-pyridin-3-yl-pyrimidin-2-ylamino)-phenyl]-amide;
- Compound 89:: 2-Methoxy-*N*-[3-(4-pyridin-4-yl-pyrimidin-2-ylamino)-phenyl]-benzamide;
- Compound 90:: 4-Trifluoromethoxy-*N*-[3-(4-pyridin-4-yl-pyrimidin-2-ylamino)-phenyl]-benzamide;
- Compound 91:: 4-Methyl-*N*-[3-(4-pyridin-4-yl-pyrimidin-2-ylamino)-phenyl]-benzamide;
and pharmaceutically active salts of these compounds.

Recent research has revealed how cells communicate with each other to coordinate the growth and maintenance of the multitude of tissues within the human body. A key element of this communication network is the transmission of a signal from the exterior of a cell to its nucleus, which results in the activation or suppression of specific genes. This process is called signal transduction.

An integral part of signal transduction is the interaction of ligands, their receptors and intracellular signal transduction molecules. Ligands are messengers that bind to specific receptors on the surface of target cells. As a result of the binding, the receptors trigger the activation of a cascade of downstream signaling molecules, thereby transmitting the message from the exterior of the cell to its nucleus. When the message reaches the nucleus, it initiates the modulation of specific genes, resulting in the production of RNA and finally proteins that carry out a specific biological function. Disturbed activity of signal transduction molecules may lead to the malfunctioning of cells and disease processes. Specifically, interference of the pathogenic PrP^{Sc} from prion diseases with neuronal cells is necessary for the prion protein to induce its neuropathological features such as neuronal vacuolization, neuronal death and gliosis with hyperastrocytosis.

A key element of this communication network is the transmission of a signal from the exterior of a cell to its nucleus, which results in the activation or suppression of specific genes. The human cellular protein kinases Abl and clk1 are two of the enzymes involved in said signal transduction process. As revealed herein said kinases Abl and clk1 serve as targets and are inhibited by the pyridylpyrimidine compounds of the general formula (I). It could be proved that prion infections and/or prion diseases can be treated and also be prevented by the inhibition of said kinase Abl using the inventive pyridylpyrimidine derivatives. Inhibition of the kinase clk1 by said pyridylpyrimidine compounds can be used for the treatment of infections and diseases.

A microarray platform technology consisting of more than 1100 signal transduction cDNAs has been established. The technology is used for the identification of changes in RNA expression patterns as a result of the manipulation of the host cell by PrP^{sc}. In addition, differential display techniques were used in order to pinpoint these changes to those enzymes which could be potential targets for drug intervention.

Employing this predefined set of signal transduction relevant cDNAs on the filters, the expression pattern of signal transduction mRNAs in neuronal mouse cells transfected with the pathogenic form of the prion protein (PrP^{Sc}) were compared with the same cells transfected with the non-pathogenic wild-type form (PrP^{c}) as a control. Interference of the PrP^{sc} with the cellular signaling events is reflected in different gene expression when compared to the control cellular situation (PrP^{c}).

Using this technology, the human cellular protein kinases FGF-R1 (also known as flg, Fl-1, Flt-2, or b-FGFR), Tkt (also known as CCK-2, DDR-2, or EDDR, EC Number 2.7.1.112), Abl (also known as c-abl), clk1, MKK7 (also known as SKK4, SAPKK4, SAPKK5, or JNKK2), LIMK-2, CaM-KI, JNK2 (also known as SAPK1a, SAPKalpha), CDC2 (also known as CDK1), PRK, the human cellular protein phosphatases PTP-SL (also known as MCP83), PTP-zeta, the cellular signal transduction molecules HSP86, and GPIR-1 were identified as potential anti-prion disease targets. Said cellular protein kinases, phosphatases and signal transduction molecules are found to be specifically up- or downregulated by PrP^{Sc} in relevant mouse neuronal cells.

Surprisingly, it was found that the following human cellular targets are significantly up- or downregulated in prion infected cells:

| target | regulation |
|---|---|
| FGF-R1 | 3.6 fold stronger |
| Abl | 5.6 fold stronger |
| MKK7 | 4.1 fold stronger |
| CDC2 | 2.0 fold weaker |
| Tkt | 2.1 fold stronger |
| LIMK-2 | 2.1 fold stronger |
| CaM-KI | 2.1 fold stronger |
| JNK2 | 2.0 fold weaker |
| PRK | 2.0 fold weaker |
| PTPzeta | 4.6 fold weaker |
| PTP-SL | 5.0 fold weaker |
| HSP86 | 4.1 fold weaker |
| GPIR-1 | 2.3 fold weaker |

Thus, one aspect of the present invention relates to a method for preventing and/or treating prion infections and/or diseases associated with said prion infections in an individual which comprises administering to the individual an amount of at least one compound of the general formula (I) and/or pharmaceutically acceptable salts thereof effective to prevent and/or treat said prion infections and/or prion diseases. Most preferred is the administration of a compound according to claim 8.

It could be proven that inhibition of one target selected from FGF-R1, Tkt, Abl, clk1, MKK7, LIMK-2, CaM-KI, JNK2, CDC2, PRK, PTP-SL, PTP-zeta, HSP86, and GPIR-1 was effective to treat prion diseases. Therefore, another aspect of the invention relates to a method for preventing and/or treating prion infections and/or prion diseases in an individual comprising the step of administering a pharmaceutically effective amount of at least one compound according of the general formula (I) and/or pharmaceutically acceptable salts thereof which inhibits at least partially the activity of one target selectef from FGF-R1, Tkt, Abl, clk1, MKK7, LIMK-2, CaM-Kl, JNK2, CDC2, PRK, PTP-SL, PTP-zeta, HSP86, and GPIR-1.

The nucleoside sequences of the genes coding for the human cellular protein kinase Abl and the protein kinase clk1 and their amino acid sequences are disclosed in form of a sequence listing shown below. The nucleoside and amino acid sequences for the kinase Abl (Accession Number: M14752) and for the kinase clk1 (Accession Numbers: XM002520, NM004071, L29222, L29219) were obtained from NCBl (National Library of Medicine: PubMed).

The compounds of general formula (I) were identified as inhibitors of at least one target selected from the group comprising FGF-R1, Tkt, Abl, clk1, MKK7, LIMK-2, CaM-Kl, JNK2, CDC2, PRK, PTP-SL, PTP-zeta, HSP86, GPIR-1 by the use of a method for detecting compounds useful for the prophylaxis and/or treatment of prion infections and/or diseases. Said method comprises
a) contacting a test compound with at least one human cellular protein kinase, phosphatase or ceiiuiar signal transduction molecule selected from the group comprising FGF-R1, Tkt, Abl, clk1, MKK7, LIMK-2, CaM-KI, JNK2, CDC2, PRK, PTP-SL, PTP-zeta, HSP86, GPIR-1; and
b) detecting the activity of said human cellular protein kinase, phosphatase or cellular signal transduction molecule.

The activity of a human cellular protein kinase, phosphatase or cellular signal transduction molecule was preferably measured by means of an enzymatic assay.

As used herein, the term "inhibitor" refers to any compound capable of downregulating, decreasing, suppressing or otherwise regulating the amount and/or activity of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1. Generally, said inhibitors, including suicide inhibitors, may be proteins, oligo- and polypeptides, nucleic acids, genes, small chemical molecules, or other chemical moieties.

The present disclosure teaches for the first time the up- or downregulation of the above-mentioned human cellular protein kinases, phosphatases, or cellular signal transduction molecules specifically involved in prion infections and/or diseases. Thus, the present invention is also directed to a method for detecting prion infections and/or diseases in an individual comprising:
a) providing a sample from said individual; and
b) adding to said sample a pharmaceutically effective amount of at least one pharmaceutically active agent; and
c) detecting activity in said sample of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, GPIR-1.

As used herein the term "sample" refers to any sample that can be taken from a living animal or human for diagnostic purposes, especially said sample comprises blood, milk, saliva, sputum, excrement, urine, spinal cord liquid, liquor, lachrymal gland liquid, biopsies and all other samples that can be taken from a living animal or human for diagnostic purposes.

The term "individual" preferably refers to mammals, especially humans or ruminants. Ruminants are, for instance, muledeer, elk, cow, cattle, sheep, goat, deer, or buffalo. Minks are an example for mammals which do not belong to the species of ruminants.

As used herein the term "ruminants" refers to an animal, for instance, cattle, sheep, goat, deer, elk, or buffalo that has four separate stomach chambers, and is therefore able to digest a wide range of organic and plant foods. The term "ruminants" refers also to exotic ruminants, like captive nyala, gemsbok, Arabian oryx, eland, kudu, scimitar-horned oryx, ankole, or bison which are also accessible to develop spongiform encephalopathy.

A similar aspect of the present invention is directed to a method for detecting prion infections and/or prion diseases in cells, cell cultures and/or cell lysates comprising:
a) providing said cells, cell cultures and/or cell lysates; and
b) adding to said cells, cell cultures and/or cell lysates a pharmaceutically effective amount of at least one pharmaceutically active agent; and
c) detecting activity in said sample of at least one human cellular protein kinase, phosphatase or cellular signal transduction molecule selected from the group comprising FGF-R1, Tkt, Abl, clk1, MKK7, LIMK-2, CaM-Kl, JNK2, CDC2, PRK, PTP-SL, PTP-zeta, HSP86, GPIR-1.

Furthermore, it has been shown that the inhibition of at least one target selected from the group comprising FGF-R1, Tkt, Abl, clk1, MKK7, LIMK-2, CaM-KI, JNK2, CDC2, PRK, PTP-SL, PTP-zeta, HSP86, GPIR-1 has an effect on the production of prions. Therefore, another aspect of the invention relates to a method for regulating the production of prions in an individual or in cells comprising the step of administering a pharmaceutically effective amount of at least one pharmaceutically active agent which inhibits at least partially the activity of at least one human cellular protein kinase, phosphatase or cellular signal transduction molecule selected from the group comprising FGF-R1, Tkt, Abl, clk1, MKK7, LIMK-2, CaM-Kl, JNK2, CDC2, PRK, PTP-SL, PTP-zeta, HSP86, GPIR-1, or which inhibits at least partially the production of at least one human cellular protein kinase, phosphatase or cellular signal transduction molecule selected from the group comprising FGF-R1, Tkt, Abl, clk1, MKK7, LIMK-2, CaM-Kl, JNK2, CDC2, PRK, PTP-SL, PTP-zeta, HSP86, GPIR-1.

The inventive compounds according to general formula (I) are examples for the above-mentioned pharmaceutically active agent. Preferably the targets FGF-R1, Tkt, Abl, clk1, MKK7, LIMK-2, CaM-KI, and CDC 2 are used with said methods.

Another type of pharmaceutically active agents useful within the methods disclosed herein are monoclonal or polyclonal antibodies which bind to a human cellular protein kinase, phosphatase or a cellular signal transduction molecule selected from the group comprising FGF-R1, Tkt, Abl, clk1, MKK7, LIMK-2, CaM-Kl, JNK2, CDC2, PRK, PTP-SL, PTP-zeta, HSP86, and GPIR-1. Thus, a further aspect of the present invention is related to said monoclonal or polyclonal antibodies which bind to a human cellular protein kinase, phosphatase or a cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1.

Another embodiment of the present invention utilizes the scientific findings that some targets such as JNK2, PRK, PTP-SL, PTP-zeta, HSP86, and GPIR-1 are downregulated during prion infection and that upregulation of the effected target by means of an activator leads to an alternative way of treating prion infections and diseases associated with prion infection.

Thus, a method was developed for regulating the production of prions either in an individual or in cells. Said methods comprise the step of administering an individual or the cells a pharmaceutically effective amount of at least one pharmaceutically active agent wherein said agent activates at least partially the activity of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1, or wherein said agent at least partially activates or stimulates the production of at least one human cellular protein kinase, phosphatase or cellular signal transduction molecule selected from the group comprising FGF-R1, Tkt, Abl, clk1, MKK7, LIMK-2, CaM-Kl, JNK2, CDC2, PRK, PTP-SL, PTP-zeta, HSP86, and GPIR-1.

Preferably the targets JNK2, PRK, PTP-SL, PTP-zeta, HSP86, and GPIR-1 are used within the above-described methods.

Because of the fact that the organism may upregulate a given target such as FGF-R1, Tkt, Abl, clk1, MKK7, LIMK-2, CaM-KI, and CDC 2 in order to compete with the prion infection, it is also a reasonable approach to further support said upregulation by means of an activator. Therefore, the above-mentioned methods apply either to targets which are downregulated but also to targets which are upregulated.

The novel and partially known pyridylpyrimidine compounds of the general formula (I) represent a new class of pharmaceuticals highly useful for the prophylaxis and treatment of prion infections and prion diseases.

Thus, a further aspect of the present invention describes the use of a compound of the general formula (I) and/or pharmaceutically acceptable salts thereof for the manufacture of a pharmaceutical formulation for prophylaxis and/or treatment of prion infections and/or diseases induced or caused by prion infection.

As used herein the Term "prion diseases" refers to transmissible spongiform encephalopathies. This group of neurologic diseases affects humans and many species of animals causing a "sponge-like" degeneration of brain tissue. Among other unique features, all of these diseases are associated with the accumulation of an abnormal form of the prion protein in nerve cells that eventually leads to the death of the host. While prion diseases can all be transmitted from one host to another, it remains contentious as to whether a virus-like infectious agent or the abnormal prion protein itself, the prion, causes the conversion of normal to abnormal protein.

Probably most mammalian species develop prion diseases. Specific examples for animals include:
- **Scrapie** sheep, goat
- **TME** (transmissible mink encephalopathy): mink
- **CWD** (chronic wasting disease): muledeer, deer, elk
- **BSE** (bovine spongiform encephalopathy): cows, cattles

Humans are also susceptible to several prion diseases. Examples are:
- **CJD** Creutzfeld-Jacob Disease
- **GSS** Gerstmann-Sträussler-Scheinker syndrome
- **FFI** Fatal familial Insomnia
- **Kuru**
- **Alpers Syndrome**

The human prion diseases include kuru, sporadic Creutzfeldt-Jakob disease (sCJD), familial CJD (fCJD), iatrogenic CJD (iCJD), Gerstmann-Sträussler-Scheinker (GSS) disease, fatal familial insomnia (FFI), and, more recently, new variant CJD (nvCJD or vCJD). In addition to these human diseases, prion-related diseases, have been recognized in several animal hosts. Scrapie is a naturally occurring disease of sheep and goats that causes ataxia, behavioral changes, and a severe pruritus that leads to scraping behavior, from which the disease was named. Additional prion diseases in animals include transmissible mink encephalopathy (TME), chronic wasting disease (CWD) of deer and elk, feline spongiform encephalopathy (FSE), and bovine spongiform encephalopathy (BSE), among others.

The transmissible nature of prion disease was first demonstrated experimentally in 1936 when Cuillé and Chelle transmitted scrapie to a healthy goat by the intraocular administration of scrapie-infected spinal cord. Thirty years later, sCJD was transmitted to chimpanzees. The pathologic feature common to all these diseases is a prominent vacuolation of the gray matter of the brain that produces a "sponge-like" appearance on light microscopy. This histopathologic appearance, coupled with the transmissible nature of these diseases, led to their collective designation as "transmissible spongiform encephalopathies" or TSEs.

The etiologic agent of the TSEs was proposed to be a "slow virus" to explain its transmissible nature and the prolonged incubation period observed during experimental transmission studies. Early experiments suggested that protein may be a critical component of the infectious agent. These studies established the basis for a new form of a transmissible pathogen, one that is composed ostensibly of only protein and lacks any replicative elements such as nucleic acid.

The term "prion" was coined to indicate an infectious agent with proteinlike properties. The unusual properties of the pathogen were demonstrated in early experiments in which conditions that degrade nucleic acids, such as exposure to ionizing and ultraviolet radiation, did not reduce the infectivity of scrapie fractions. On the other hand, treatments that degrade protein, such as prolonged exposure to proteases, correlated with a reduction in infectivity. A protein with relative resistance to protease digestion was found to be consistently present in the brains of animals and humans with TSE. Surprisingly, this protein was found to be one that is normally encoded by a chromosomal gene of the host.

Thus, the question raised, how a normally expressed protein could also be a transmissible pathogen? It was hypothesized and later demonstrated that PrP exists in two major isoforms: the nonpathogenic or cellular form, designated PrP^{c}, and the pathogenic or scrapie-inducing form, designated PrP^{Sc}. Both PrP^{c} and PrP^{Sc} have the same amino acid sequence, yet they differ in their biochemical properties: PrP^{c} is soluble in nondenaturing detergents and completely degraded by proteases, whereas PrP^{Sc} is insoluble in nondenaturing detergents and shows a relative resistance to proteases. Structural studies of PrP^{c} and PrP^{Sc} indicate a difference in the conformation of the two isoforms: PrP^{c} is predominantly helical, whereas PrP^{Sc} contains at least 40% pleated sheet structure. Conversion to this sheet structure appears to be the fundamental event in prion disease. The ultimate mechanism of how cells die coincident with the generation of prions is still unclear. Simple accumulation of pathogenic protein may not be sufficient to explain disease, however, it may constitute a critical step in cellular dysfunction.

It was shown that the pyridylpyrimidine compounds of the general formula (I) are highly effective for the prophylaxis and/or treatment of prion infections and/or prion diseases selected from the group comprising Scrapie, TME, CWD, BSE, CJD, vCJD, GSS, FFI, Kuru, and Alpers Syndrome. Preferably, the pyridylpyrimidine derivatives are used for preventing and/or treating BSE, vCJD, or CJD.

The above-mentioned prion infections and/or diseases associated with prion infections can be treated using the inventive pyridylpyrimidine derivatives by targeting at least one of the human cellular protein kinases, phosphatases or cellular signal transduction molecules selected from the group comprising FGF-R1, Tkt, Abl, clk1, MKK7, LIMK-2, CaM-KI, JNK2, CDC2, PRK, PTP-SL, PTP-zeta, HSP86, GPIR-1. Thereby, the compounds according to general formula (I) act as inhibitors for at least one of the above-mentioned targets and especially as inhibitors for at least one enzyme selected from the group comprising FGF-R1, Tkt, Abl, clk1, MKK7, LIMK-2, CaM-KI, and CDC 2.

According to these findings a further aspect of the present invention is directed to a method for preventing and/or treating prion infections and/or prion diseases in an individual comprising the step of administering a pharmaceutically effective amount of at least one phamiaceutically active agent which inhibits at least partially the activity of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, GPIR-1, or which inhibits at least partially the production of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, GPIR-1.

Another aspect is related to a method for preventing and/or treating prion infections and/or prion diseases in cells or cell cultures comprising the step of administering a pharmaceutically effective amount of at least one pharmaceutically active agent which inhibits at least partially the activity of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, GPIR-1, or which inhibits at least partially the production of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, GPIR-1.

The inventive pyridylpyrimidine compounds of formula (I) are examples for the above-mentioned inhibitor. Said pyridylpyrimidine compounds and/or pharmaceutically acceptable salts thereof are administered in a dosage corresponding to an effective concentration in the range of 0.01 - 50 µM, preferably in the range of 0.01 - 10 µM, more preferably in the range of 0.01 - 1 µM, and most preferably in the range of 0.01 - 0.1 µM.

Because of the fact that the targets JNK2, PRK, PTP-SL, PTP-zeta, HSP86, and GPIR-1 are downregulated in cells infected with prions, an upregulation of said targets represents another strategy in order to treat prion infections and diseases like CJD (nvCJD or vCJD) associated with prion infections. Said upregulation can be performed by activators.

An agent that is able to upregulate, increase, activate, or stimulate the activity of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1, but especially of JNK2, PRK, PTP-SL, PTP-zeta, HSP86, and GPIR-1 is named "activator".

Thus, another embodiment of the present invention describes a method for preventing and/or treating prion infections and/or diseases in an individual comprising the step of administering a pharmaceutically effective amount of at least one pharmaceutically active agent which activates at least partially the activity of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1, or which activates or stimulates the production of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1. Preferably, said method is directed to the targets JNK2, PRK, PTP-SL, PTP-zeta, HSP86, and GPIR-1.

As used herein, the term "agent" or "pharmaceutically active agent" refers to any chemical compound capable of down- or upregulating, de- or increasing, suppressing, activation, stimulating or otherwise regulating the amount and/or activity of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1. Generally, said agents may be proteins, oligo- and polypeptides, nucleic acids, genes, aptamers, small chemical molecules, or other chemical moieties. An agent may be either an inhibitor or an activator and especially an inhibitor for the enzymes FGF-R1, Tkt, Abl, clk1, MKK7, LIMK-2, CaM-Kl, and CDC 2 and an activator for the targets JNK2, PRK, PTP-SL, PTP-zeta, HSP86, and GPIR-1.

One special kind of said pharmaceutically active agents are aptamers which function as regulators of the activity of a wide range of cellular molecules such as human cellular protein kinase and phosphatase. Aptamers are nucleic acid molecules selected in vitro to bind small molecules, peptides, or proteins with high affinity and specificity. Aptamers not only exhibit highly specific molecular recognition properties but are also able to modulate the function of their cognate targets in a highly specific manner by agonistic or antagonistic mechanisms. Most famous examples for aptamers are DNA aptamers or RNA aptamers.

Further examples for pharmaceutically active agents are the pyridylpyrimidine compounds of the present invention and/or pharmaceutically acceptable salts thereof. Said compounds are administered in a dosage corresponding to an effective concentration in the range of 0.01 - 50 µM, preferably in the range of 0.01 - 10 µM, more preferably in the range of 0.01 - 1 µM, and most preferably in the range of 0.01 - 0.1 µM.

The compounds of general formula (I) can be administered in a daily dosage in the range of 25 mg to 1000 mg, preferably in a daily dosage of 400 mg to 600 mg, more preferably in a daily dosage of 500 mg, and most preferably in continuously increased daily dosages starting at a initial daily dosage of 400 mg and ending up in a daily dosage of 600 mg at the end of the treatment.

A question is how PrP^{c} does convert to PrP^{Sc}? Potential mechanisms that initiate conversion of PrP^{c} to PrP^{Sc} include a germ line mutation of the human prion protein gene (PRNP), a somatic mutation within a particular neuron, and spontaneous conversion of PrP^{c} to an aberrant conformation that is not refolded appropriately to its native structure. The prion protein gene (PRNP) is the single gene on the short arm of chromosome 20 in humans which encodes the normal cellular isoform of the prion protein. Regardless of the initiating event, once an "infectious unit" has been generated, PrP^{Sc} appears to act as a conformational template by which PrP^{c} is converted to a new molecule of PrP^{Sc} through protein-protein interaction of PrP^{Sc} and PrP^{c}. This concept is supported by several studies which show that mice with the normal PrP gene deleted (PrP knockout mice) do not develop prion disease after inoculation with scrapie. Furthermore, transgenic (Tg) mice that express a chimeric PrP gene made of human and mouse segments develop protease-resistant chimeric mouse-human PrP^{sc} in their brains when inoculated with brain extracts from humans with prion disease. These findings clearly illustrate that prions do not self-replicate but instead convert nonpathogenic PrP^{c} to pathogenic PrP^{Sc}.

In its sporadic or nonfamilial form, **CJD** is the most common of the human prion diseases. Confusion and forgetfulness which progress rapidly to severe cortical dementia in combination with ataxia, myoclonus, and an abnormal electroencephalogram (EEG) represents the "classic tetrad" of CJD. However, a host of other neurologic signs and symptoms, including diffuse or focal weakness, painful neuropathy, chore-iform movements, hallucinations, cortical blindness, primary language disturbance, supranuclear ophthalmoplegia, and alien hand syndrome, among others, have been observed. As the disease progresses from the early stage, ataxia commonly limits the patient's mobility.

**Familial CJD** (fCJD) includes those cases with a dominantly inherited mutation of the PRNP gene, in which the pathologic features of spongiform change occur in the absence of GSS-type plaques. Although, familial cases of CJD tend to have a clinical and pathologic phenotype similar to that of sCJD.

The original description of a patient with the onset of ataxia and dysarthria followed by variable degrees of pyramidal and extrapyramidal symptoms and late developing dementia defines the classic presentation of GSS. The duration of said disease ranges from 2 to 10 years. Death usually results from secondary infection, often from aspiration pneumonia because of impaired swallowing. The presence of plaque deposits regionally or diffusely throughout the cortex that are immunoreactive to anti-human PrP antibodies is the hallmark of this form of prion disease.

FFI is a genetic disorder which manifests itself by many symptoms due to the degeneration of a certain part of the brain, the thalamus. The affected area of the brain is the area responsible for sleep, the thalamus. The thalamus is the center which communications from the brain to the body and the body to the brain pass through for proper directions to where a signal should be received. When sleep takes place, it is thought that the thalamus becomes less efficient at this signal transfer function allowing for the vegetative state of sleep to come over an individual. Consequently, the symptoms of fatal familial insomnia are directly related to the malfunction of the responsibilities of the thalamus, namely sleep.

There are four stages of the disease before an individual's life ends. The first stage is progressive insomnia, the characteristic feature of fatal familial insomnia. By now, there is no cure for this illness.

The term "familial" means: affecting several members of the same family, usually as a result of an underlying genetic mutation.

The occurrence of **vCJD** is sobering because it appears to represent a situation in which the prion has "jumped" species, in this case from cow to human. Because the pathologic features and clinical presentation of vCJD differ significantly from those of sCJD, it is considered a new "strain" of human prion disease. The same "protein signature" was observed following experimental transmission of BSE to several animal hosts, supporting the idea that vCJD results from the infection of humans with BSE. vCJD occurs primarily in younger individuals (average age 27) with a somewhat protracted course of approximately 16 months. The brain shows diffuse vacuolation and the presence of distinctive dense core PrP-containing plaques surrounded by a halo of spongiform change.

Kuru is the condition which first brought prion diseases to prominence in the 1950s. The disease was found in geographically isolated tribes in New Guinea. It was established that ingesting brain tissue of dead relatives for religious reasons was likely to be the route of transmission.

**Alpers Syndrome** is the name given to prion diseases in infants.

**Scrapie** is the accepted, albeit somewhat colloquial, name for the naturally occurring transmissible spongiform encephalopathy of sheep and goats found worldwide. Scrapie also infects laboratory mice and hamsters making it one of the most important sources of new scientific information about this group of disorders. Scrapie was the first example of this type of disease to be noticed and has been known about for many hundreds of years. There are two possible methods of transmission in sheep: a) Infection of pasture with placental tissue carrying the agent followed by ingestion, or b) direct sheep-lamb transmission.

**CWD** is a fatal neurodegenerative disease of deer and elk, now known to be a transmissible spongiform encephalopathy. To date, affected animals have been found exclusively in the United States.

### BSE

Bovine spongiform encephalopathy or "mad cow disease" appears to have originated from scrapie that has been recognized in Europe since the mid-18th century. It has since spread to most sheep-breeding countries and is widespread in the United Kingdom, where until 1988 the rendered carcasses of livestock (including sheep) were fed to ruminants and other animals as a protein-rich nutritional supplement.

During rendering, carcasses from which all consumable parts had been removed were milled and then decomposed in large vats by boiling at atmospheric or higher pressures, producing an aqueous slurry of protein under a layer of fat (tallow). After the fat was removed, the slurry was desiccated into a meat and bone meal product that was packaged by the animal food industry and distributed to owners of livestock and other captive animals (e.g., zoo and laboratory animals, breeding species, pets).

A further aspect is related to a method for regulating the expression of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1 in an individual comprising the step of administering the individual a pharmaceutically effective amount of at least one pharmaceutically active agent wherein said agent inhibits at least partially the transcription of DNA or the translation of RNA.

And a still further aspect of the present invention relates to a method for regulating the expression of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1 in the cells, the method comprising the step of administering the cells a pharmaceutically effective amount of at least one pharmaceutically active agent wherein said agent inhibits at least partially the transcription of DNA or the translation of RNA.

As used herein, the term "regulating expression and/or activity" generally refers to any process that functions to control or modulate the quantity or activity (functionality) of a cellular component. Static regulation maintains expression and/or activity at some given level. Upregulation refers to a relative increase in expression and/or activity. Accordingly downregulation refers to a relative decrease in expression and/or activity. Downregulation is synonymous with inhibition of a given cellular component's activity.

The transcription of DNA and the translation of RNA can be inhibited by oligonucleotides or oligonucleotide derivatives. Thus, the present invention discloses oligonucleotides and derivatives of oligonucleotides which may be used in the above-mentioned methods. The oligonucleotide and/or its derivatives bind to the DNA and/or RNA encoding a human cellular protein kinase, phosphatase or a cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1 and suppress the transcription of DNA or translation of RNA.

As described above, said prion infection and/or disease associated with said prion infection is selected from the group comprising Scrapie, TME, CWD, BSE, vCJD, CJD, GSS, FFI, Kuru, and Alpers Syndrome. Preferably, the method is used for prophylaxis and/or treatment of BSE, vCJD, or CJD. The above disclosed methods are preferably applied to CJD, vCJD, and BSE, more preferably applied to vCJD and BSE, and most perferably applied to BSE.

Some methods of the present invention identify compounds useful for prophylaxis and/or treatment of prion infections and/or diseases by screening a test compound, or a library of test compounds, for its ability to inhibit at least one of the above-mentioned human cellular protein kinases, phosphatases, or cellular signal transduction molecules, identified herein as characteristically up- or downregulated during prion production or growth inside a cell or individual. A variety of assay protocols and detection techniques are well known in the art and easily adapted for this purpose by a skilled practitioner. Such methods include, but are not limited to, high throughput assays (e.g., microarray technology, phage display technology), and *in vitro* and *in vivo* cellular and tissue assays.

Thus, a solid support is disclosed in the present invention useful for screening compounds useful for the prophylaxis and/or treatment of prion infections and/or diseases in an individual, the solid support comprising at least one immobilized oligonucleotide, wherein said oligonucleotide encodes one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1.

A further aspect of the present invention is related to a solid support useful for screening compounds useful for the prophylaxis and/or treatment of prion infections and/or diseases in an individual, the solid support comprising at least one immobilized human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1.

In another embodiment, a component of the above-mentioned methods comprises peptide fragments of one or more of the above-identified human cellular protein kinases, phosphatases or cellular signal transduction molecules immobilized on a solid support. Once again the most preferred solid support embodiment would contain polymers of sufficient quality and quantity to detect all of the above-mentioned human cellular protein kinases, phosphatase and cellular signal transduction molecules (e.g., a nucleic acid or a peptide microarray). A variety of supports and constructions of the same for the methods disclosed herein are well known in the art and easily adapted for this purpose by a skilled practitioner (cf., for example: Marschall, 1999 "Do-it-yourself gene watching" Science 286, 444-447; Service 2000 "Protein arrays step out of DNA's shadow" Science 289, 1673).

It is preferred that mRNA is measured as an indication of expression. Methods for assaying for mRNA include, but are not limited to, Northern blots, slot blots, dot blots, and hybridization to an ordered array of oligonucleotides. Nucleic acid probes useful for assay of a sample are preferably of sufficient length to specifically hybridize only to appropriate, complementary transcripts. Typically the oligonucleotide probes will be at least 10 to 25 nucleotides in length. In some cases longer probes of at least 30 to 50 nucleotides will be desirable.

The cDNA oligonucleotides immobilized on said membrane filter which are used for detecting the up- or downregulation of the above-mentioned human cellular protein kinases, phosphatases, and cellular signal transduction molecules by hybridization to the radioactively labeled cDNA probes have the nucleotide sequences listed in table 1.

**Table 1: Nucleotide sequences of cDNA-arrays**

| Cellular kinase, phosphatase, or signal transduction molecule | Sequence of immobilized DNA on arrays (in relation to the respective Acc No) |
|---|---|
| FGF-R1 | 41 bp - 2619bp (X52833) |
| Tkt (EC 2.7.1.112) | 1 bp - 3096bp (X74764) |
| Abl | 2153 bp - 3765 bp (M14752) |
| clk1 | 156 bp -1610 bp (L29219) |
| MKK7 | 77 bp - 1323 bp (AF013588) |
| CDC2 | 77 bp - 1050 bp (X05360) |
| CaMKI | 145 bp - 1452 bp (L41816) |
| JNK2 | 507 bp - 1782 bp (L31951) |
| LIMK-2 | 963 bp - 2047 bp (D45906) |
| PRK | n.a bp - 1862 bp (U56998) |
| PTP zeta (EC 3.1.3.48) | 148 bp - 7604 bp (X54135) |
| PTP-SL | 862 bp - 1902 bp (NM 002849) |
| HSP86 | n.a bp - n.a bp (X07270) |
| GPIR-1 | n.a bp - n.a bp (n.a) |

Tkt has been assigned to the EC Number: 2.7.1.112
PTP zeta has been assigned to the EC Number: 3.1.3.48

The nucleoside sequences of the genes coding for the human cellular protein kinases, phosphatases, or cellular signal transduction molecules listed in Table 1 together with the amino acid sequences and the enzyme commission numbers (E.C. numbers) of said enzymes can be obtained from NCBI (National Library of Medicine: PubMed; Web address: www.ncbi.nlm.nih.gov/entrez).

The polypeptide product of gene expression may be assayed to determine the amount of expression as well. Methods for assaying for a protein include, but are not limited to, western blot, immuno-precipitation, radioimmuno assay, and peptide immobilization in an ordered array. It is understood, however, that any method for specifically and quantitatively measuring a specific protein or mRNA product can be used.

A variety of supports upon which nucleic acids or peptides can be immobilized are known in the art, for example filters, or polyvinyl chloride dishes. Any solid surface to which oligonucleotides or peptides can be bound, either directly or indirectly, either covalently or non-covalently, can be used. A preferred solid support is a microarray membrane filter or a "biochip". These contain particular polymer probes in predetermined locations on the array. Each predetermined location may contain more than one molecule of the probe, but each molecule within the predetermined location has an identical sequence.

The present invention incorporates by reference in their entirety techniques well known in the field of molecular biology. These techniques include, but are not limited to, techniques described in the following publications:
Ausubei, F.M. et al. eds., "Short Protocols In Molecular Biology" 4th Ed. 1999, John Wiley & Sons, NY (ISBN 0-471-32938-X);
Old, R.W. & S.B. Primrose "Principles of Gene Manipulation: An Introduction To Genetic Engineering" 3rd Ed. 1985, Blackwell Scientific Publications, Boston. Studies in Microbiology: V.2, 409 pp. (ISBN 0-632-01318-4);
Mayer, R.J. & J.H. Walker eds. "Immunochemical Methods In Cell and Molecular Biology" 1987, Academic Press, London. 325 pp. (ISBN 0-12480-855-7);
Winnacker, E.L. "From Genes To Clones: Introduction To Gene Technology" 1987 VCH Publishers, NY. (translated by Horst lbelgaufts) 634 pp. (ISBN 0-89573-614-4).

As described above, a microarray platform technology was developed consisting of more than 1100 signal transduction cDNAs immobilized on a solid support. Thus, another aspect of the present invention is directed to a solid support useful for detecting prion infections and/or diseases in an individual, the solid support comprising an immobilized oligonucleotide, wherein said oligonucleotide is capable of detecting activity of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1.

The present invention discloses also for the first time a solid support useful for detecting prion infections and/or diseases in cells, the solid support comprising an immobilized oligonucleotide, wherein said oligonucleotide is capable of detecting activity of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1.

The present invention further incorporates by reference in their entirety techniques well known in the field of microarray construction and analysis. These techniques include, but are not limited to, techniques described in the following patents and patent applications describing array of biopolymeric compounds and methods for their fabrication:
U.S. Pat. Nos. 5,807,522; 6,087,102; WO 93/17126; WO 95/11995;
WO 95/35505; EP 742 287; and EP 799 897.

Techniques also include, but are not limited to, techniques described in the following patents and patent application describing methods of using arrays in various applications:
U.S. Pat. Nos. 5,994,076; 6,033,860; 6,040,138; 6,040,140;
WO 95/21265; WO 96/31622; WO 97/10365; WO 97/27317;
EP 373 203; and EP 785 280

Still a further aspect of the present invention is directed to pharmaceutical compositions comprising at least one pharmaceutically active agent together with a pharmaceutically acceptable carrier, excipient or diluents. Examples for pharmaceutically active agents are the above-mentioned inventive compounds according to formula (I), or other small chemical molecules, antibodies, aptamers, oligo- and polynucleotides, genes and other biological components capable of regulating the activity of at least one target selected from the group comprising FGF-R1, Tkt, Abl, clk1, MKK7, LIMK-2, CaM-KI, JNK2, CDC2, PRK, PTP-SL, PTP-zeta, HSP86, and GPIR-1, or which are effective to treat prion infections and diseases associated with prion infection. Said prion infections and diseases are preferably Scrapie, TME, CWD, BSE, vCJD, CJD, GSS, FFI, Kuru, and Alpers Syndrome.

Thus, the pharmaceutical compositions according to the present invention may comprise an inhibitor, such as the inventive pyridylpyrimidine compounds or an activator such as aptamers for at least one target selected from FGF-R1, Tkt, Abl, clk1, MKK7, LIMK-2, CaM-KI, JNK2, CDC2, PRK, PTP-SL, PTP-zeta, HSP86, and GPIR-1. It is also possible to have a combination of inhibitors or activators as active ingredients in one single pharmaceutical composition. Furthermore, suitable are also combinations of at least one inhibitor and at least one activator for different targets within a single pharmaceutical composition. For example, a pharmaceutical composition could comprise compound 12 as an inhibitor for, for instance, the target Abl, and an activator such as an aptamer for, for instance, the human cellular protein kinase JNK2.

Said pharmaceutical compositions are useful for the prophylaxis and/or treatment of an individual afflicted with prions comprising at least one agent capable of inhibiting and/or activating at least partially the activity, the expression, and/or the production of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1.

The pyridylpyrimidine compounds of the present invention are basic and form pharmaceutically acceptable salts with organic and inorganic acids. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, d-o-tolyltartaric acid, tartronic acid, α-toluic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

It is also possible to obtain acid addition salts with amino acids like methionine, tryptophane, lysine or arginine, especially with pyridylpyrimidine compounds of the general formula (I) carrying a carboxylic acid residue.

Depending upon the substituents on the inventive pyridylpyrimidine compounds, one may be able to form salts with bases, too. Thus, for example, if there are carboxylic acid substituents in the molecule, salts may be formed with inorganic as well as organic bases such as, for example, NaOH, KOH, NH₄OH, tetraalkylammonium hydroxide, and the like.

The compounds of the general formula (I) can also be administered in form of their pharmaceutically active salts optionally using substantially nontoxic pharmaceutically acceptable carriers, excipients or diluents. The medications of the present invention are prepared in a conventional solid or liquid carrier or diluents and a conventional pharmaceutically-made adjuvant at suitable dosage level in a known way. The preferred preparations are in administratable form which is suitable for oral application. These administratable forms, for example, include pills, tablets, film tablets, coated tablets, capsules, powders and deposits.

The preferred administratable forms are tablets, film tablets, coated tablets, gelatin capsules, and opaque capsules. Each pharmaceutical composition contains at least one compound of the general formula (I), preferably compound 53 and/or pharmaceutically acceptable salts thereof in an amount of 50 mg to 150 mg, preferably 80 mg to 120 mg, and most preferably in an amount of 100 mg per formulation.

Furthermore, the subject of the present invention also includes pharmaceutical preparations for parenteral, including dermal, intradermal, intragastrical, intracutaneous, intravasal, intravenous, intramuscular, intraperitoneal, intranasal, intravaginal, intrabuccal, percutaneous, rectal, subcutaneous, sublingual, topical or transdermal application, which in addition to typical vehicles and diluents contain a pyridylpyrimidine compound of the general formula (I) and/or a pharmaceutically acceptable salt thereof as active ingredient.

Within the disclosed methods the pharmaceutical compositions of the present invention, containing pyridylpyrimidine derivatives of the general formula (I) as active ingredients, will typically be administered in admixture with suitable carrier materials selected with respect to the intended form of administration, i.e. oral tablets, capsules (either solid-filled, semi-solid filled or liquid filled), powders for constitution, oral gels, elixirs, dispersible granules, syrups, suspensions, and the like, and consistent with conventional pharmaceutical practices. For example, for oral administration in the form of tablets or capsules, the active drug component may be combined with any oral nontoxic pharmaceutically acceptable inert carrier, such as lactose, starch, sucrose, cellulose, magnesium stearate, dicalcium phosphate, calcium sulfate, talc, mannitol, ethyl alcohol (liquid forms) and the like. Moreover, when desired or needed, suitable binders, lubricants, disintegrating agents and coloring agents may also be incorporated in the mixture. Powders and tablets may be comprised of from about 5 to about 95 percent inventive composition.

Suitable binders include starch, gelatin, natural sugars, corn sweeteners, natural and synthetic gums such as acacia, sodium alginate, carboxymethylcellulose, polyethylene glycol and waxes. Among the lubricants, there may be mentioned for use in these dosage forms, boric acid, sodium benzoate, sodium acetate, sodium chloride, and the like. Disintegrants include starch, methylcellulose, guar gum and the like. Sweetening and flavoring agents and preservatives may also be included where appropriate. Some of the terms noted above, namely disintegrants, diluents, lubricants, binders and the like, are discussed in more detail below.

Additionally, the compositions of the present invention may be formulated in sustained release form to provide the rate controlled release of any one or more of the components or active ingredients to optimize the therapeutic effects, i.e. antihistaminic activity and the like. Suitable dosage forms for sustained release include layered tablets containing layers of varying disintegration rates or controlled release polymeric matrices impregnated with the active components and shaped in tablet form or capsules containing such impregnated or encapsulated porous polymeric matrices.

Liquid form preparations include solutions, suspensions and emulsions. As an example may be mentioned water or water-propylene glycol solutions for parenteral injections or addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier such as inert compressed gas, e.g. nitrogen.

For preparing suppositories, a low melting wax such as a mixture of fatty acid glycerides such as cocoa butter is first melted, and the active ingredient is dispersed homogeneously therein by stirring or similar mixing. The molten homogeneous mixture is then poured into convenient sized molds, allowed to cool and thereby solidifies.

Also included are solid form preparations which are intended to be converted, shortly before use, to liquid form preparations for either oral or parenteral administration. Such liquid forms include solutions, suspensions and emulsions.

The inventive pyridylpyrimidine compounds of the present invention may also be deliverable transdermally. The transdermal compositions may take the form of creams, lotions, aerosols and/or emulsions and can be included in a transdermal patch of the matrix or reservoir type as are conventional in the art for this purpose.

The term capsule refers to a special container or enclosure made of methyl cellulose, polyvinyl alcohols, or denatured gelatins or starch for holding or containing compositions comprising the active ingredients. Hard shell capsules are typically made of blends of relatively high gel strength bone and pork skin gelatins. The capsule itself may contain small amounts of dyes, opaquing agents, plasticizers and preservatives.

Tablet means compressed or molded solid dosage form containing the active ingredients with suitable diluents. The tablet can be prepared by compression of mixtures or granulations obtained by wet granulation, dry granulation or by compaction well known to a person skilled in the art.

Oral gels refers to the active ingredients dispersed or solubilized in a hydrophillic semi-solid matrix.

Powders for constitution refers to powder blends containing the active ingredients and suitable diluents which can be suspended in water or juices.

Suitable diluents are substances that usually make up the major portion of the composition or dosage form. Suitable diluents include sugars such as lactose, sucrose, mannitol and sorbitol, starches derived from wheat, corn rice and potato, and celluloses such as microcrystalline cellulose. The amount of diluents in the composition can range from about 5 to about 95% by weight of the total composition, preferably from about 25 to about 75%, more preferably from about 30 to about 60% by weight.

The term disintegrants refers to materials added to the composition to help it break apart (disintegrate) and release the medicaments. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses and cross-linked microcrystalline celluloses such as sodium croscarmellose, alginates such as alginic acid and sodium alginate, clays such as bentonites, and effervescent mixtures. The amount of disintegrant in the composition can range from about 2 to about 20% by weight of the composition, more preferably from about 5 to about 10% by weight.

Binders characterize substances that bind or "glue" powders together and make them cohesive by forming granules, thus serving as the "adhesive" in the formulation. Binders add cohesive strength already available in the diluents or bulking agent. Suitable binders include sugars such as sucrose, starches derived from wheat, corn rice and potato; natural gums such as acacia, gelatin and tragacanth; derivatives of seaweed such as alginic acid, sodium alginate and ammonium calcium alginate; cellulosic materials such as methylcellulose and sodium carboxymethylcellulose and hydroxypropylmethylcellulose; polyvinylpyrrolidone; and inorganics such as magnesium aluminum silicate. The amount of binder in the composition can range from about 2 to about 20% by weight of the composition, more preferably from about 3 to about 10% by weight, even more preferably from about 3 to about 6% by weight.

Lubricant refers to a substance added to the dosage form to enable the tablet, granules, etc. after it has been compressed, to release from the mold or die by reducing friction or wear. Suitable lubricants include metallic stearates such as magnesium stearate, calcium stearate or potassium stearate; stearic acid; high melting point waxes; and water soluble lubricants such as sodium chloride, sodium benzoate, sodium acetate, sodium oleate, polyethylene glycols and D,L-leucine. Lubricants are usually added at the very last step before compression, since they must be present on the surfaces of the granules and in between them and the parts of the tablet press. The amount of lubricant in the composition can range from about 0.2 to about 5% by weight of the composition, preferably from about 0.5 to about 2%, more preferably from about 0.3 to about 1.5% by weight.

Glidents are materials that prevent caking and improve the flow characteristics of granulations, so that flow is smooth and uniform. Suitable glidents include silicon dioxide and talc. The amount of glident in the composition can range from about 0.1% to about 5% by weight of the total composition, preferably from about 0.5 to about 2% by weight.

Coloring agents are excipients that provide coloration to the composition or the dosage form. Such excipients can include food grade dyes and food grade dyes adsorbed onto a suitable adsorbent such as clay or aluminum oxide. The amount of the coloring agent can vary from about 0.1 to about 5% by weight of the composition, preferably from about 0.1 to about 1%.

As used herein, a "pharmaceutically effective amount" of an inhibitor and/or an activator is an amount effective to achieve the desired physiological result, either in cells treated *in vitro* or in a subject treated *in vivo.* Specifically, a pharmaceutically effective amount is an amount sufficient to inhibit and or activate, for some period of time, one or more of the clinically defined pathological processes associated with the prion infection. The effective amount may vary depending on the specific inhibitor and/or activator selected, and is also dependent on a variety of factors and conditions related to the subject to be treated and the severity of the infection. For example, if an inhibitor and/or activator is to be administered *in vivo,* factors such as the age, weight and health of the patient as well as dose response curves and toxicity data obtained in pre-clinical animal work would be among those considered. If the inhibitor and/or activator is to be contacted with the cells *in vitro,* one would also design a variety of pre-clinical *in vitro* studies to assess such parameters as uptake, half-life, dose, toxicity, etc. The determination of a pharmaceutically effective amount for a given pharmaceutically active agent is well within the ability of those skilled in the art.

It is also apparent to a person skilled in the art that detection includes any method known in the art useful to indicate the presence, absence, or amount of a detection target. Such methods may include, but are not limited to, any molecular or cellular techniques, used singularly or in combination, including, but not limited to: hybridization and/or binding techniques, including blotting techniques and immunoassays; labeling techniques (chemiluminescent, colorimetric, fluorescent, radioisotopic); spectroscopic techniques; separations technology, including precipitations, electrophoresis, chromatography, centrifugation, ultrafiltration, cell sorting; and enzymatic manipulations (e.g., digestion).

It should be stressed that all above-mentioned features, aspects, and details of the present invention discussed and described in connection with infections and infectious diseases, equally apply to neurodegenerative diseases, like Alzheimer.

It is readily apparent to those skilled in the art that other suitable modifications and adaptations of the compositions and methods of the invention described herein are evident and may be made without departing from the scope of the invention or the embodiments disclosed herein. Having now described the present invention in detail, the same will be more clearly understood by reference to the following examples, which are included for purposes of illustration only and are not intended to be limiting of the invention.

### Description of figures

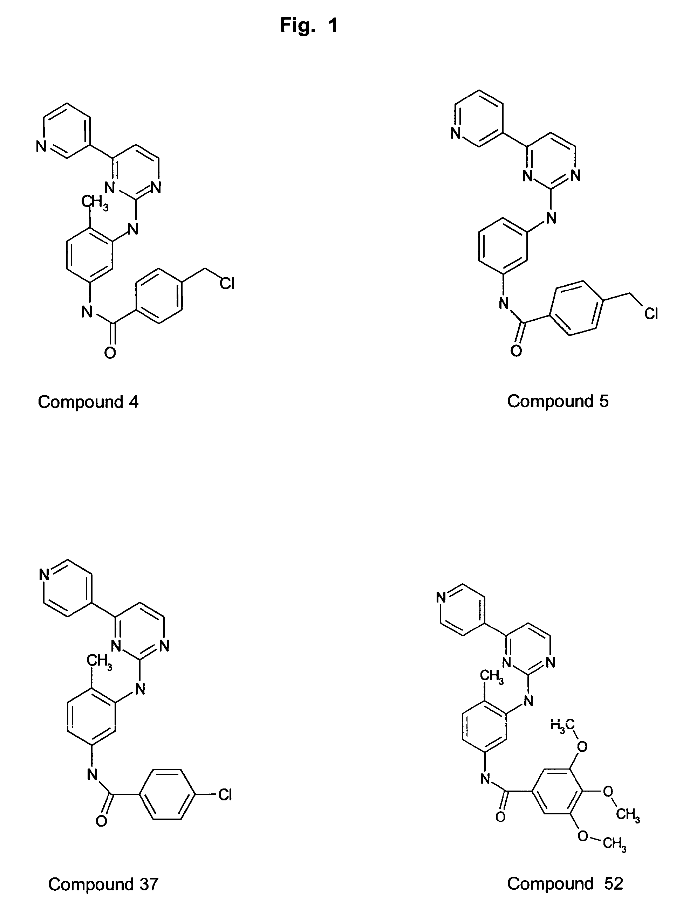

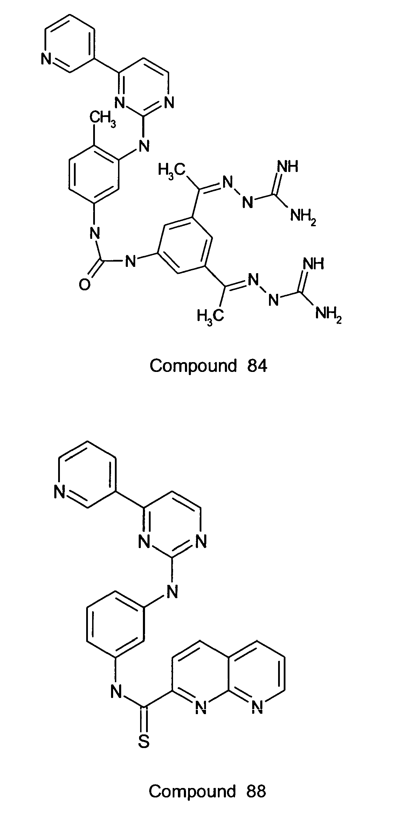

- Fig. 1: shows 6 selected pyridylpyrimidine derivatives which are suitable inhibitors for prion diseases, namely compounds 4, 5, 37, 52, 84, and 88;
- Fig. 2: shows the compound 4-(4-Methylpiperazin-1-ylmethyl)-*N*-[4-methyl-3-(4-pyridin-3-yl-pyrimidin-2-ylamino)-phenyl]-benzamide, also known as Gleevec™;
- Fig. 3: shows selected compounds that have been identified as potent inhibitors in a prion propagation assay at a concentration of 5 µm.

### Examples

### Materials and methods

### 1. Generation of cDNA-arrays on membranes

In order to manufacture cDNAs-arrays on membranes, the following strategy was pursued: cDNAs encoding parts of or full length proteins of interest - in the following referred to as "target cDNAs" - were cloned into the plasmid Bluescript II KS⁺ (Stratagene, USA). Large scale purifications of these plasmids were performed according to standard techniques and 200 µl aliquots (1 µg/µl plasmid concentration) were transferred into appropriate 96well plates. Plates were closed with sealing tape and chilled on ice for 5 minutes after incubation for 10 minutes at 95°C. 10 µl of 0.6 N NaOH were added and the mix was stored for 20 minutes at room temperature before addition of 10 µl 2.5 M Tris-HCl pH 7.1 and 20 µl 40x SSC (3 M NaCl, 300 mM Sodium Citrate, pH 7.0). Target cDNAs were spotted onto Nylon or Nitrocellulose membranes using a BioGrid (BioRobotics, UK) equipped with a 0.7 mm pintool. In this way, between 200 ng and 350 ng of plasmids encoding target cDNAs were transferred onto the membranes and crosslinked to the membranes by ultraviolet light (1.2x10⁵ µJ/cm²). The arrays were stored for use in subsequent experiments at room temperature.

### 2. Generation of cells

PrP^{Sc}- and PrP^{c}-transfected mouse neuronal cells (N2A) were cultured in MEM (Minimum Essential Medium, Life Technologies) supplemented with 10% fetal calf serum at 37°C and 5% CO₂ to obtain ~6x10⁶ cells per tissue culture flask.

### 3. Lysis of cells, isolation of total RNA and purification of polyA⁺ RNA

After incubation of the cells with the virus for the respective time-points, cells were washed twice with phosphate buffered saline (PBS) and then trypsinized. Subsequently, cells were removed from the culture dish by resuspension with PBS. Afterwards, cells were sedimented and directly lysed in Tri reagent by repetitive pipetting using in 1ml of Tri reagent (Molecular Research Centre, Inc., USA) per 1x10⁶ cells.

The lysates were stored at room temperature for 5 minutes and then centrifuged at 12000xg for 15 minutes at 4°C. The supernatant was mixed with 0,1 ml of 1-bromo-3-chloropropane per 1 ml of Tri reagent and vigorously shaken. The suspension was stored for 5 minutes at room temperature and then centrifuged at 12000xg for 15 minutes at 4°C.

The colourless upper phase was transferred into new tubes, mixed with 5 µl of poly-acryl-carrier (Molecular Research Centre, Inc., USA) and with 0.5 ml of isopropanol per 1 ml of Tri reagent and vigorously shaken. The samples were stored at room temperature for 5 minutes and then centrifuged at 12000xg for 8 minutes at 4°C. The supernatant was removed and the RNA pellet washed twice with 1 ml of 75% ethanol. The pellet was dried and resuspended for 10 minutes at 55°C in 50 µl of RNase-free buffer (5 mM Tris-HCl pH 7.5). The integrity of the isolated RNA was determined by agarose/formaldehyde gel electrophoresis and the RNA was finally stored at -70°C for use in subsequent experiments.

### 4. Preparation of radioactively labelled cDNA probes from RNA

In order to obtain radioactively labelled cDNA probes total RNA was transcribed into a cDNA-probe in the presence of radioactively labelled dATP. 12 µl bidestilled DEPC (Diethylpyrocarbonate) treated H₂O containing 0.5 µg of primer TXN (5'-TTT TTT TTT TTT TTT TXN-3' with T → dTTP; N → dATP, dCTP, dGTP or dTTP; X → dATP, dCTP or dGTP) and total RNA (1 to 10 µg) were shaken between 5 and 15' at 60°C and then incubated on ice for 2 minutes. After centrifugation (30 seconds, 10000xg) 7 µl of a mix consisting of 100 µCi dATP-P³³ (Amersham, UK) which were dried under vacuum previously and resuspended in 4 µl first strand buffer (Life Technologies, USA), 2 µl 0.1M DTT (Dithiothreitol) and 1 µl labelling solution (4 mM dCTP, dGTP, dTTP each and 80 µM dATP final concentration) were added. Following the addition of 1 µl Superscript II reverse transcriptase (Life Technologies, USA) the reaction was incubated for 10 minutes at room temperature and then for 60 minutes at 38°C. Subsequently, the reaction was vigorously shaken for 30 minutes at 68°C after adding 5 µl 0.5 M EDTA and 25 µl 0.6M NaOH.

Unincorporated nucleotides were removed from the labelling reaction using ProbeQuant G-50 columns (Amersham, UK). The column was vigorously shaken and centrifuged for 1 minute at 735xg in an appropriate reaction tube after bottom closure and lid were removed. The column was placed into a new reaction tube, the probe was applied onto the centre of the column material and the column was centrifuged for 2 minutes at 735xg. The flow-trough was transferred into new reaction tubes and filled up to a volume of 100 µl with bidestilled H₂O. The probe was precipitated by centrifugation for 15 minutes at 12000xg after 4 µl 5M NaCl, 1 µl poly-acryl-carrier (Molecular Research Centre, Inc., USA) and 250 µl ethanol were added. The supernatant was discarded and the pellet was dried at 50°C for 5 minutes before starting with the hybridisation.

### 5. Hybridisation of radioactively labelled cDNA-probes to cDNA-arrays

The pellet was resuspended in 10 µl C₀T DNA (1 µg/µl, Roche Diagnostics, Germany), 10 µl yeast tRNA (1µg/µl Sigma, USA) and 10 µl polyA (1 µg/µl, Roche Diagnostics, Germany) and incubated at 55°C for 5 minutes. Herring sperm DNA was added to a final concentration of 100 µg/ml and the volume was filled up to 100 µl with 5 µl 10% SDS (Sodiumdodecylsulfat), 25 µl 20x SSPE (3M Sodium chloride, 0,2 M Sodium dihydrogen phosphate monohydrate, 0,02 M Ethylenedinitrilo tetraacetic acid, disodium salt dihydrate; pH 7,4) and bidestilled H₂O. The mix was put on 95°C for 5 minutes, centrifuged for 30 seconds at 10000xg and vigorously shaken for 60 minutes at 65°C. A 1 µl aliquot of the probe was used to measure the incorporation of radioactive dATP with a scintillation counter. Probes with at least a total of 20x10⁶ cpm were used.

The arrays were prehybridised for at least 3 hours at 42°C in hybridisation solution in a roller bottle oven. After prehybridization the radioactively labelled probe was added into the hybridisation solution and hybridisation was continued for 20 to 40 hours.

The probe was discarded and replaced with wash solution A (2xSSC). The arrays were washed twice in wash solution A at room temperature in the roller oven. Afterwards, wash solution A was replaced by wash solution B (2x SSC, 0.5% SDS) preheated to 65°C and arrays were washed twice for 30 minutes at 65°C. Then, wash solution B was replaced by wash solution C (0.5x SSC, 0.5% SDS) preheated to 65°C and arrays were washed twice for 30 minutes at 65°C. The moist arrays were wrapped in airtight bags and exposed for 8 to 72 hours on erased phosphoimager screens (Fujifilm, Japan).

### 6. Analysis of cDNA-arrays

The exposed phosphoimager screens were scanned with a resolution of 100µ and 16bits per pixel using a BAS-1800 (Fujifilm, Japan). Files were imported into the computer program ArrayVision (Imaging Research, Canada). Using the program's features, the hybridization signals of each target cDNA were converted into numbers. The strength of the hybridization signals reflected the quantity of RNA molecules present in the probe. Differentially expressed genes were selected according to the ratio of their signal strength after normalization to the overall intensity of the arrays.

### 7. Cell culture and expression of 3F4-tagged PrP (3F4-ScN2a)

The mouse neuroblastoma cell line 3F4-ScN2a represents a stably transfected clone of ScN2a cells (PrP^{Sc} infected N2a cells) which overexpress 3F4-epitope-tagged murine PrP. Residues 109 and 112 of murine PrP were replaced by methionine to introduce the epitope for reactivity with the monoclonal anti-PrP antibody 3F4. Cells were maintained in Dulbecco's modified Eagle's (DMEM) or Opti-MEM medium containing 10 % fetal calf serum, antibiotics and glutamin. For generation of stable transfectants we used the vector pcDNA3.1/Zeo (Invitrogen; Leek, The Netherlands). Lipofection of cells with recombinant plasmids was done using standard procedures and recombinant clones were selected by addition of 300 µg Zeocin/ml medium.

### 8. Treatment of cells with inhibitors

All tested compounds were solubilized in DMSO (dimethylsulfoxide), and prepared as 10 mM stock solutions. The drugs were applied to the cells described above for three days in final concentrations between 5 and 20 µM.

### 9. lmmunoblot and proteinase K (PK) analysis

Confluent cell cultures were lysed in cold lysis buffer (10 mM Tris-HCl, pH 7.5; 100 mM NaCl; 10 mM EDTA; 0.5 % Triton X-100; 0.5 % DOC) (EDTA: ethylene diamine tetraacetate; Triton X-100: t-octylphenoxypolyethoxyethanol; DOC: deoxycholic acid). Postnuclear lysates were split between those with and without proteinase K digestion. Samples without proteinase K digestion were supplemented with proteinase inhibitors (5 mM PMSF, 0.5 mM Pefabloc, and aprotinin) (PMSF: phenylmethylsulfonyl fluoride) and directly precipitated with ethanol. Samples for proteinase K digestion were incubated with 20 µg/ml proteinase K for 30 min at 37°C; digestion was stopped with proteinase inhibitors, and samples were ethanol precipitated. After centrifuging for 30 min at 3,500 rpm the pellets were redissolved in TNE buffer (10 mM Tris-HCl pH7.5, 100 mM NaCl, 1mM EDTA) and gel loading buffer was then added. After boiling for 5 min an aliquot was analyzed on 12.5 % PAGE. For Western blot analysis, the proteins were electrotransferred to PVDF membranes (polyvinylidendifluorid). The membrane was blocked with 5 % non-fat dry milk in TBST (0.05 % Tween 20, 100 mM NaCl, 10 mM Tris-HCl, pH 7.8) (Tween 20: polyoxyethylenesorbitan monolaurate; Tris-HCl: Tris-(hydroxymethyl)-aminomethane-hydrochloride), incubated overnight with the primary antibody at 4°C and stained using the enhanced chemiluminescence blotting kit from Amersham Corporation. Specific immuno-staining of the PrP^{c} and PrP^{Sc} forms were obtained with the prion protein specific antibody 3F4 (Signet Pathologies, U.S.A.).

### 10. Results

Determination of the amount of the pathogenic form of the prion protein PrP^{Sc} upon treatment of prion infected cells with different types of small molecule protein kinase inhibitors resulted in the identification of a compound class of pyridylpyrimidine derivatives examplified by the compound 4-(4-Methylpiperazin-1-ylmethyl)-*N*-[4-methyl-3-(4-pyridin-3-yl-pyrimidin-2-ylamino)-phenyl]-benzamide (compound 53) and compounds 4, 5, and 37.

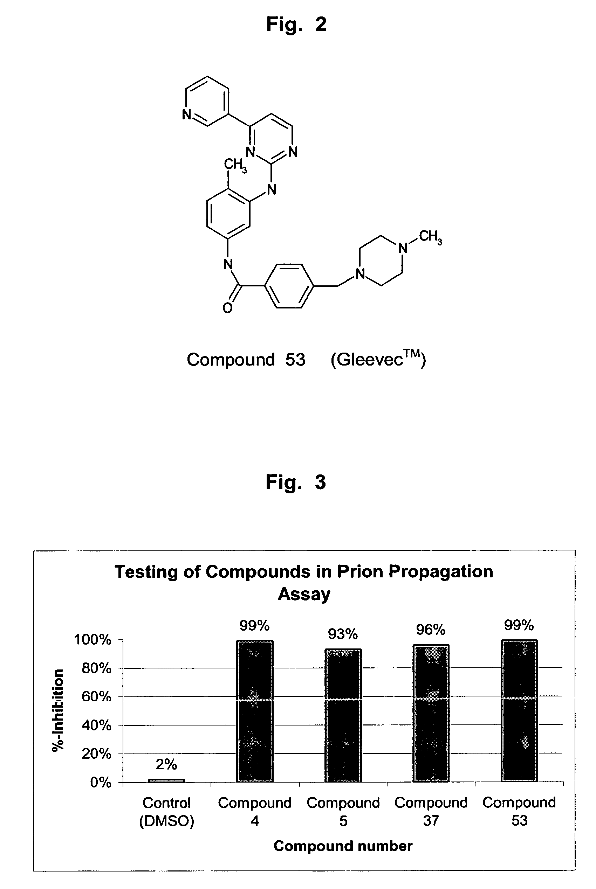

These compounds significantly reduced the amount of PrP^{Sc} in prion infected cells in a concentration range between 5 and 20 µM (final concentration). As shown in Fig. 3 the selected compounds 4, 5, 37, and 53 inhibit almost completely the activity of prion propagation within said concentration range.

The compounds did not show any toxic effects on the cells in these concentrations. Therefore these molecules described herein serve as potential inhibitors for the medical intervention of prion diseases such as transmissible spongiform encephalitis (TSE) infections which include Bovine spongiform encephalitis (BSE) or the new variant of Creutzfeld Jakob disease (vCJK).

## Claims

1. Compounds having the general formula (I): 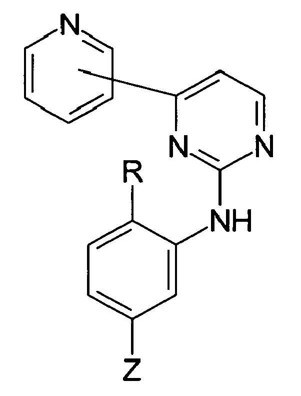 wherein:
R represents hydrogen or methyl;
Y, Y', Y" are independently of each other ―H, ―F, ―Cl, ―Br, ―I, ―CH₂F, ―CH₂Cl, ―CH₂Br, ―CH₂l, ―OH, ―OCH₃, ―CH₃, ―CN, ―OCF₃, 4-methylpiperazin-1-yl-methyl, ―C(CH₃)=N―NH―C(NH)―NH₂
Z represents ―NO₂, ―NH₂, ―NH―CO―X, ―NH―CS―X, ―NH―CO―NH―X, ―NH-SO₂―X;
X represents thiophenyl, cyclohexyl, isoquinolinyl, naphthyl, quinolinyl, cyclopentyl, pyridinyl, naphthyridinyl, or 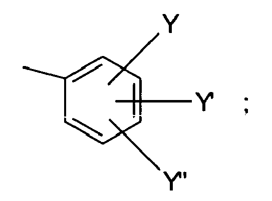 and pharmaceutically acceptable salts thereof.

2. Use of a compound having the general formula (I): 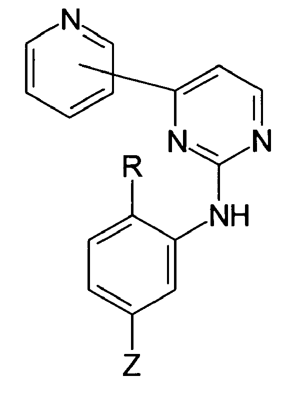 wherein:
R represents hydrogen or methyl;
Y, Y', Y" are independently of each other -H, -F, -Cl, -Br, -I, -CH₂F, -CH₂Cl, -CH₂Br, -CH₂l, -OH, -OCH₃, -CH₃, -CN, -OCF₃, 4-methylpiperazin-1-yl-methyl, -C(CH₃)=N-NH-C(NH)-NH₂;
Z represents -NO₂, -NH₂, -NH-CO-X, -NH-CS-X, -NH-CO-NH-X, -NH-SO₂-X;
X represents thiophenyl, cyclohexyl, isoquinolinyl, naphthyl, quinolinyl, cyclopentyl, pyridinyl, naphthyridinyl, or 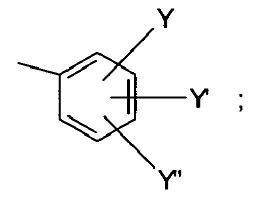 and pharmaceutically acceptable salts thereof as pharmaceutically active agents.

3. Use of a compound having the general formula (I): 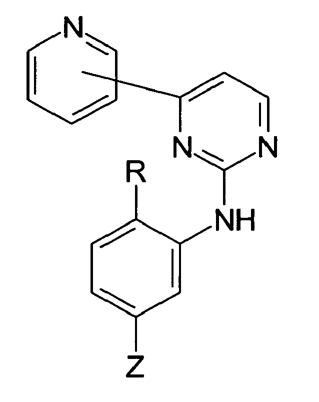 wherein:
R represents hydrogen or methyl;
Y, Y', Y" are independently of each other -H, -F, -Cl, -Br, -I, -CH₂F, -CH₂Cl, -CH₂Br, -CH₂l, -OH, -OCH₃, -CH₃, -CN, -OCF₃, 4-methylpiperazin-1-yl-methyl, -C(CH₃)=N-NH-C(NH)-NH₂;
Z represents -NO₂, -NH₂, -NH-CO-X, -NH-CS-X, -NH-CO-NH-X, -NH-SO₂-X;
X represents thiophenyl, cyclohexyl, isoquinolinyl, naphthyl, quinolinyl, cyclopentyl, pyridinyl, naphthyridinyl, or 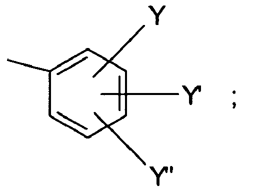 and pharmaceutically acceptable salts thereof for prophylaxis and/or treatment of infectious diseases or neurodegenerative diseases.

4. Use of a compound according to claim 2 or 3 for the prophylaxis and/or treatment of prion infections and/or diseases induced by prion infection.

5. Use of a compound according to any one of claims 2 - 4 wherein R represents hydrogen.

6. Use of a compound according to any one of claims 2 - 5 wherein Z represents ―NH―CO―X or ―NH―SO₂―X.

7. Use of a compound according to any one of claims 2 - 6 wherein Y, Y', Y" are independently of each other -H, -F, -Cl, -CH₂F, -CH₂Cl, -OH, -OCH₃, -CH₃, -CN, -OCF₃, 4-methylpiperazin-1-yl-methyl.

8. Use of a compound according to claim 2 or 3 wherein the compound is selected from the group comprising:
(3-Nitrophenyl)-(4-pyridin-3-yl-pyrimidin-2-yl)-amine;
(3-Aminophenyl)-(4-pyridin-3-yl-pyrimidin-2-yl)-amine;
(5-Amino-2-methylphenyl)-(4-pyridin-3-yl-pyrimidin-2-yl)-amine;
4-Chloromethyl-*N*-[4-methyl-3-(4-pyridin-3-yl-pyrimidin-2-ylamino)-phenyl]-benzamide;
4-Chloromethyl-*N*-[3-(4-pyddin-3-yl-pydmidin-2-ylamino)-phenyl]-benzamide;
4-(4-Methylpiperazin-1-ylmethyl)-*N*-[3-(4-pyridin-3-yl-pyrimidin-2-ylamino)-phenyl]-benzamide;
Thiophene-3-carboxylic acid [4-methyl-3-(4-pyridin-4-yl-pyrimidin-2-ylamino)-phenyl]-amide;
4-Chloro-*N*-[4-methyl-3-(4-pyridin-3-yl-pyrimidin-2-ylamino)-phenyl]-benzamide;
4-Chloro-*N*-[3-(4-pyridin-3-yl-pyrimidin-2-ylamino)-phenyl]-benzamide;
3,4,5-Trimethoxy-*N*-[3-(4-pyridin-3-yl-pyrimidin-2-ylamino)-phenyl]-benzamide;
4-Cyano-*N*-[3-(4-pyridin-3-yl-pyrimidin-2-ylamino)-phenyl]-benzamide;
4-Methoxy-*N*-[3-(4-pyridin-3-yl-pyrimidin-2-ylamino)-phenyl]-benzamide;
4-Chloro-*N*-[3-(4-pyridin-3-yl-pyrimidin-2-ylamino)-phenyl]-benzenesulfonamide;
Thiophene-3-carboxylic acid [3-(4-pyridin-3-yl-pyrimidin-2-ylamino)-phenyl]-amide;
3,5-Dimethoxy-*N*-[3-(4-pyridin-3-yl-pyrimidin-2-ylamino)-phenyl]-benzamide;
3,4,5-Trimethoxy-*N*-[4-methyl-3-(4-pyridin-3-yl-pyrimidin-2-ylamino)-phenyl]-benzamide;
4-Cyano-*N*-[4-methyl-3-(4-pyridin-3-yl-pyrimidin-2-ylamino)-phenyl]-benzamide;
4-Methoxy-*N*-[4-methyl-3-(4-pyridin-3-yl-pyrimidin-2-ylamino)-phenyl]-benzamide;
4-Chloro-*N*-[4-methyl-3-(4-pyridin-3-yl-pyrimidin-2-ylamino)-phenyl]-benzenesulfonamide;
Thiophene-3-carboxylic acid [4-methyl-3-(4-pyridin-3-yl-pyrimidin-2-ylamino)-phenyl]-amide;
3,5-Dimethoxy-*N*-[4-methyl-3-(4-pyridin-3-yl-pyrimidin-2-ylamino)-phenyl]-benzamide;
4-Trifluoromethoxy-*N*-[4-methyl-3-(4-pyridin-3-yl-pyrimidin-2-ylamino)-phenyl]-benzamide;
Cyclohexanecarboxylic acid [4-methyl-3-(4-pyridin-3-yl-pyrimidin-2-ylamino)-phenyl]-amide;
Cyclohexanecarboxylic acid [3-(4-pyridin-3-yl-pyrimidin-2-ylamino)-phenyl]-amide;
Isoquinoline-5-sulfonic acid [4-methyl-3-(4-pyridin-3-yl-pyrimidin-2-ylamino)-phenyl]-amide;
Isoquinoline-5-sulfonic acid [3-(4-pyridin-3-yl-pyrimidin-2-ylamino)-phenyl]-amide;
(5-Nitro-2-methylphenyl)-(4-pyridin-2-yl-pyrimidin-2-yl)-amine;
(5-Amino-2-methylphenyl)-(4-pyridin-2-yl-pyrimidin-2-yl)-amine;
3,4,5-Trimethoxy-*N*-[4-methyl-3-(4-pyridin-2-yl-pyrimidin-2-ylamino)-phenyl]-benzamide;
4-Cyano-*N*-[4-methyl-3-(4-pyridin-2-yl-pyrimidin-2-ylamino)-phenyl]-benzamide;
(3-Aminophenyl)-(4-pyridin-2-yl-pyrimidin-2-yl)-amine;
4-Chloro-*N*-[4-methyl-3-(4-pyridin-2-yl-pyrimidin-2-ylamino)-phenyl]-benzamide;
Cyclohexanecarboxylic acid [4-methyl-3-(4-pyridin-2-yl-pyrimidin-2-ylamino)-phenyl]-amide;
4-Cyano-*N*-[4-methyl-3-(4-pyridin-4-yl-pyrimidin-2-ylamino)-phenyl]-benzamide;
4-Chloro-*N*-[4-methyl-3-(4-pyridin-4-yl-pyrimidin-2-ylamino)-phenyl]-benzenesulfonamide;
4-Methoxy-*N*-[4-methyl-3-(4-pyridin-4-yl-pyrimidin-2-ylamino)-phenyl]-benzamide;
4-Chloro-*N*-[4-methyl-3-(4-pyridin-4-yl-pyrimidin-2-ylamino)-phenyl]-benzamide;
Cyclohexanecarboxylic acid [3-(4-pyridin-4-yl-pyrimidin-2-ylamino)-phenyl]-amide;
3,5-Dimethoxy-N-[3-(4-pyridin-4-yl-pyrimidin-2-ylamino)-phenyl]-benzamide;
(5-Amino-2-methylphenyl)-(4-pyridin-4-yl-pyrimidin-2-yl)-amine;
Thiophene-3-carboxylic acid [3-(4-pyridin-4-yl-pyrimidin-2-ylamino)-phenyl]-amide;
4-Chloro-*N*-[3-(4-pyridin-4-yl-pyrimidin-2-ylamino)-phenyl]-benzenesulfonamide;
4-Chloro-*N*-[3-(4-pyridin-4-yl-pyrimidin-2-ylamino)-phenyl]-benzamide;
(3-Aminophenyl)-(4-pyridin-4-yl-pyrimidin-2-yl)-amine;
(3-Nitrophenyl)-(4-pyridin-4-yl-pyrimidin-2-yl)-amine;
4-Trifluoromethoxy-*N*-[4-methyl-3-(4-pyridin-4-yl-pyrimidin-2-ylamino)-phenyl]-benzamide;
Isoquinoline-5-sulfonic acid [3-(4-pyridin-4-yl-pyrimidin-2-ylamino)-phenyl]-amide;
4-Methoxy-*N*-[3-(4-pyridin-4-yl-pyrimidin-2-ylamino)-phenyl]-benzamide;
4-Cyano-*N*-[3-(4-pyridin-4-yl-pyrimidin-2-ylamino)-phenyl]-benzamide;
3,4,5-Trimethoxy-*N*-[3-(4-pyridin-4-yl-pyrimidin-2-ylamino)-phenyl]-benzamide;
3,5-Dimethoxy-*N*-[4-methyl-3-(4-pyridin-4-yl-pyrimidin-2-ylamino)-phenyl]-benzamide;
3,4,5-Trimethoxy-*N*-[4-methyl-3-(4-pyridin-4-yl-pyrimidin-2-ylamino)-phenyl]-benzamide;
4-(4-Methylpiperazin-1-ylmethyl)-*N*-[4-methyl-3-(4-pyridin-3-yl-pyrimidin-2-ylamino)-phenyl]-benzamide;
4-Methyl-*N*-[4-methyl-3-(4-pyridin-2-yl-pyrimidin-2-ylamino)-phenyl]-benzenesulfonamide;
4-Methoxy-*N*-[4-methyl-3-(4-pyridin-2-yl-pyrimidin-2-ylamino)-phenyl]-benzamide;
3,5-Dimethoxy-*N*-[4-methyl-3-(4-pyridin-2-yl-pyrimidin-2-ylamino)-phenyl]-benzamide;
Naphthalene-2-carboxylic acid [4-methyl-3-(4-pyridin-3-yl-pyrimidin-2-ylamino)-phenyl]-amide;
*N*-[3-(4-Pyridin-4-yl-pyrimidin-2-ylamino)-phenyl]-benzamide;
4-Chloro-*N*-[3-(4-pyridin-2-yl-pyrimidin-2-ylamino)-phenyl]-benzamide;
4-Methoxy-*N*-[3-(4-pyridin-2-yl-pyrimidin-2-ylamino)-phenyl]-benzamide;
4-Chloro-*N*-[3-(4-pyridin-2-yl-pyrimidin-2-ylamino)-phenyl]-benzenesulfonamide;
Thiophene-2-carboxylic acid 3-(4-pyridin-2-yl-pyrimidin-2-yl-amino)-phenyl]-amide;
Naphthalene-2-sulfonic-acid [3-(4-pyridin-2-yl-pyrimidin-2-yl-amino)-phenyl]-amide;
Isoquinoline-5-sulfonic-acid [3-(4-pyridin-2-yl-pyrimidin-2-yl-amino)-phenyl]-amide;
Cylopentanecarboxylic acid 3-(4-pyridin-2-yl-pyrimidin-2-yl-amino)-phenyl]-amide;
Naphthalene-2-carboxylic acid [3-(4-pyridin-2-yl-pyrimidin-2-ylamino)-phenyl]-amide;
4-Cyano-N-[3-(4-pyridin-2-yl-pyrimidin-2-ylamino)-phenyl]-benzamide;
3,5-Dimethoxy-*N*-[3-(4-pyridin-2-yl-pyrimidin-2-ylamino)-phenyl]-benzamide;
4-Bromo-*N*-[3-(4-pyridin-2-yl-pyrimidin-2-ylamino)-phenyl]-benzamide;
4-Methyl-*N*-[3-(4-pyridin-2-yl-pyrimidin-2-ylamino)-phenyl]-benzamide;
4-Fluoro-*N*-3-(4-pyridin-2-yl-pyrimidin-2-ylamino)-phenyl]-benzenesulfonamide;
3,5-Dichloro-*N-*[3-(4-pyridin-2-yl-pyrimidin-2-ylamino)-phenyl]-benzamide;
*N*-[3-(4-Pyridin-2-yl-pyrimidin-2-ylamino)-phenyl]-benzamide;
4-Chloromethyl-*N*-[3-(4-pyridin-2-yl-pyrimidin-2-ylamino)-phenyl]-benzamide;
4-Methyl-*N*-3-(4-pyridin-2-yl-pyrimidin-2-ylamino)-phenyl]-benzenesulfonamide;
4-(4-Methylpiperazin-1-ylmethyl)-*N*-[3-(4-pyridin-2-yl-pyrimidin-2-ylamino)-phenyl]-benzamide;
Naphthalene-2-carboxylic acid [3-(4-pyridin-4-yl-pyrimidin-2-ylamino)-phenyl]-amide;
2-Methoxy-*N*-[4-methyl-3-(4-pyridin-3-yl-pyrimidin-2-ylamino)-phenyl]-benzamide;
2-Methoxy-*N*-[3-(4-pyridin-3-yl-pyrimidin-2-ylamino)-phenylJ-benzamide;
4-Methyl-*N*-[3-(4-pyridin-3-yl-pyrimidin-2-ylamino)-phenyl]-benzamide;
4-Methyl-*N*-[4-methyl-3-(4-pyridin-3-yl-pyrimidin-2-ylamino)-phenyl]-benzamide;
*N*-[4-Methyl-3-(4-pyridin-3-yl-pyrimidin-2-ylamino)-phenyl]-benzamide;
1-(3,5-Diacetyl-phenyl)-3-[4-methyl-3-(4-pyridin-3-yl-pyrimidin-2-ylamino)-phenyl]-urea;
1-{3,5-Bis-(amidinohydrazone)-phenyl }-3-[4-methyl-3-(4-pyridin-3-yl-pyrimidin-2-ylamino)-phenyl]-urea;
*N*-[4-Methyl-3-(4-pyridin-3-yl-pyrimidin-2-ylamino)-phenyl]-nicotinamide;
*N*-[3-(4-Pyridin-3-yl-pyrimidin-2-ylamino)-phenyl]-nicotinamide;
[1,8]Naphthyridine-2-carboxylic acid [3-(4-pyridin-3-yl-pyrimidin -2-ylamino)-phenyl]-amide;
[1,8]Naphthyridine-2-carbothioic acid [3-(4-pyridin-3-yl-pyrimi-din-2-ylamino)-phenyl]-amide;
2-Methoxy-*N*-[3-(4-pyridin-4-yl-pyrimidin-2-ylamino)-phenyl]-benzamide;
4-Trifluoromethoxy-*N*-[3-(4-pyridin-4-yl-pyrimidin-2-ylamino)-phenyl]-benzamide;
4-Methyl-*N*-[3-(4-pyridin-4-yl-pyrimidin-2-ylamino)-phenyl]-benzamide;
and/or a pharmaceutically acceptable salt of these compounds.

9. Use according to claim 8 wherein the compound is 4-(4-Methylpiperazin-1-ylmethyl)-*N*-[4-methyl-3-(4-pyridin-3-yl-pyrimidin-2-ylamino)-phenyl]-benzamide.

10. Use of a compound recited in any one of claims 2 - 9 and/or pharmaceutically acceptable salts thereof for the manufacture of a pharmaceutical composition for prophylaxis and/or treatment of prion infections and/or diseases induced by prion infection and/or neurodegenerative diseases.

11. Use according to claim 4 or 10 wherein said prion infection and/or disease is selected from the group comprising Scrapie, TME, CWD, BSE, CJD, vCJD, GSS, FFI, Kuru, and Alpers Syndrome.

12. Use according to claim 11 wherein said prion infection is BSE, vCJD, or CJD.

13. Use of a compound recited in any one of claims 2 - 9 as an inhibitor for at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, GPIR-1.

14. Use of a compound according to any one of claims 2 to 13 wherein the compound of the general formula (I) and/or pharmaceutically acceptable salts thereof is administered in a dosage corresponding to an effective concentration in the range of 0.01 - 50 µM.

15. Pharmaceutical composition comprising at least one compound recited in any one of claims 2 - 9 as an active ingredient, together with one or more pharmaceutically acceptable carrier(s), excipient(s) or diluents.

16. Method for preventing and/or treating infections and/or diseases in an individual which comprises administering to the individual an amount of at least one compound recited in claims 2 - 9 and/or pharmaceutically acceptable salts thereof effective to prevent and/or treat said infections and/or diseases.

17. Method for preventing and/or treating prion infections and/or prion diseases induced by prion infections in an individual which comprises administering to the individual an amount of at least one compound recited in any one of claims 3 to 8 and/or pharmaceutically acceptable salts thereof effective to prevent and/or treat said prion infection and/or disease.

18. Method for preventing and/or treating prion infections and/or prion diseases induced by prion infections in an individual which comprises administering to the individual an amount of at least one compound recited in claim 8 and/or pharmaceutically acceptable salts thereof effective to prevent and/or treat said prion infection and/or disease.

19. Method for detecting prion infections and/or prion diseases in an individual comprising:
a) providing a sample from said individual;
b) adding to said sample a pharmaceutically effective amount of at least one pharmaceutically active agent; and
c) detecting activity in said sample of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, GPIR-1.

20. Method according to claim 19 wherein said sample comprises blood, milk, saliva, sputum, excrement, urine, spinal cord liquid, liquor, lachrymal gland liquid, biopsies and all other samples that can be taken from a living animal or human for diagnostic purposes.

21. Method for detecting prion infections and/or prion diseases in cells, cell cultures and/or cell lysates comprising:
a) providing said cells, cell cultures and/or cell lysates;
b) adding to said cells, cell cultures and/or cell lysates a pharmaceutically effective amount of at least one pharmaceutically active agent; and
c) detecting activity in said sample of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, GPIR-1.

22. Method for preventing and/or treating prion infections and/or prion diseases in an individual comprising the step of administering a pharmaceutically effective amount of at least one pharmaceutically active agent which inhibits at least partially the activity of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, GPIR-1, or which inhibits at least partially the production of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, GPIR-1.

23. Method for preventing and/or treating prion infections and/or prion diseases in cell or cell cultures comprising the step of administering a pharmaceutically effective amount of at least one pharmaceutically active agent which inhibits at least partially the activity of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, GPIR-1, or which inhibits at least partially the production of at least one human cellular protein kinase, phosphatase or cellular signal transduction molecule selected from the group comprising FGF-R1, Tkt, Abi, clk1, MKK7, LIMK-2, CaM-KI, JNK2, CDC2, PRK, PTP-SL, PTP-zeta, HSP86, GPIR-1.

24. Method for regulating the production of prions in an individual comprising the step of administering a pharmaceutically effective amount of at least one pharmaceutically active agent which inhibits at least partially the activity of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, GPIR-1, or which inhibits at least partially the production of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, GPIR-1.

25. Method for regulating the production of prions in cells comprising the step of administering a pharmaceutically effective amount of at least one pharmaceutically active agent which inhibits at least partially the activity of at least one human cellular protein kinase, phosphatase or cellular signal transduction molecule selected from the group comprising FGF-R1, Tkt, Abl, clk1, MKK7, LIMK-2, CaM-Kl, JNK2, CDC2, PRK, PTP-SL, PTP-zeta, HSP86, GPIR-1, or which inhibits at least partially the production of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, GPIR-1.

26. A monoclonal or polyclonal antibody that binds to a human cellular protein kinase, phosphatase or a cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1.

27. Method according to any one of claims 19 - 25, wherein the agent is a monoclonal or polyclonal antibody which binds to a human cellular protein kinase, phosphatase or a cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1.

28. Method according to any one of claims 19 - 25, wherein the agent is at least one compound of the general formula (I) and/or pharmaceutically acceptable salts thereof.

29. Method according to any one of claims 16 - 25, wherein the agent is 4-(4-Methylpiperazin-1-ylmethyl)-*N*-[4-methyl-3-(4-pyridin-3-yl-pyrimidin-2-yl-amino)-phenyl]-benzamide and/or pharmaceutically acceptable salts thereof.

30. Method according to claim 28 wherein the compound of the general formula (I) and/or pharmaceutically acceptable salts thereof is administered in a dosage corresponding to an effective concentration in the range of 0.01 - 50 µM.

31. Method for detecting compounds useful for the prophylaxis and/or treatment of prion infections and/or diseases comprising:
a) contacting a test compound with at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, GPIR-1; and
b) detecting the activity of said human cellular protein kinase, phosphatase or cellular signal transduction molecule.

32. Method for preventing and/or treating prion infections and/or diseases in an individual comprising the step of administering a pharmaceutically effective amount of at least one pharmaceutically active agent which activates at least partially the activity of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1, or which activates or stimulates the production of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1.

33. Method for regulating the production of prions in an individual comprising the step of administering an individual a pharmaceutically effective amount of at least one pharmaceutically active agent wherein said agent activates at least partially the activity of at least one human cellular protein kinase, phosphatase or ceiiuiar signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1, or wherein said agent at least partially activates or stimulates the production of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1.

34. Method for regulating the production of prions in cells comprising the step of administering the cells a pharmaceutically effective amount of at least one pharmaceutically active agent wherein said agent activates at least partially the activity of at least one human cellular protein kinase, phosphatase or cellular signal transduction molecule selected from the group comprising FGF-R1, Tkt, Abl, clk1, MKK7, LIMK-2, CaM-Kl, JNK2, CDC2, PRK, PTP-SL, PTP-zeta, HSP86, and GPIR-1, or wherein said agent at least partially activates or stimulates the production of at least one human cellular protein kinase, phosphatase or cellular signal transduction molecule selected from the group comprising FGF-R1, Tkt, Abl, clk1, MKK7, LIMK-2, CaM-Kl, JNK2, CDC2, PRK, PTP-SL, PTP-zeta, HSP86, and GPIR-1 in the cells.

35. Method for regulating the expression of at least one human cellular protein kinase, phosphatase or cellular signal transduction molecule selected from the group comprising FGF-R1, Tkt, Abl, clk1, MKK7, LIMK-2, CaM-Kl, JNK2, CDC2, PRK, PTP-SL, PTP-zeta, HSP86, and GPIR-1 in an individual comprising the step of administering the individual a pharmaceutically effective amount of at least one pharmaceutically active agent wherein said agent inhibits at least partially the transcription of DNA or the translation of RNA.

36. Method for regulating the expression of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1 in the cells comprising the step of administering the cells a pharmaceutically effective amount of at least one pharmaceutically active agent wherein said agent inhibits at least partially the transcription of DNA or the translation of RNA.

37. Oligonucleotide that binds to the DNA or RNA encoding a human cellular protein kinase, phosphatase or a cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1.

38. Method according to claim 22, 23, 24, 25, 35 or 36 wherein the agent is a oligonucleotide which binds to the DNA and/or RNA encoding a human cellular protein kinase, phosphatase or a cellular signal transduction molecule selected from the group comprising FGF-R1, Tkt, Abl, clk1, MKK7, LIMK-2, CaM-Kl, JNK2, CDC2, PRK, PTP-SL, PTP-zeta, HSP86, and GPIR-1.

39. Method according to claims 16, 17, 18, 19, 22, 24, 32, 33, or 35 wherein said individual is a human or ruminant.

40. Method according to any one of claims 17, 18, 19, 21, 22, 23, 31, or 32 wherein said prion infection and/or prion disease is selected from the group comprising Scrapie, TME, CWD, BSE, vCJD, CJD, GSS, FFI, Kuru, and Alpers Syndrome.

41. Method according to claim 40 wherein said prion infection and/or prion disease is BSE, vCJD, or CJD.

42. A solid support useful for detecting prion infections and/or diseases in an individual, the solid support comprising an immobilized oligonucleotide, wherein said oligonucleotide is capable of detecting activity of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1.

43. A solid support useful for detecting prion infections and/or diseases in cells, the solid support comprising an immobilized oligonucleotide, wherein said oligonucleotide is capable of detecting activity of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1.

44. A solid support useful for screening compounds useful for the prophylaxis and/or treatment of prion infections and/or diseases in an individual, the solid support comprising at least one immobilized oligonucleotide, wherein said oligonucleotide encodes one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1.

45. A solid support useful for screening compounds useful for the prophylaxis and/or treatment of prion infections and/or diseases in an individual, the solid support comprising at least one immobilized human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1.

46. Composition useful for the prophylaxis and/or treatment of an individual afflicted with prions comprising at least one agent capable of inhibiting at least partially the activity of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1.

47. Composition useful for the prophylaxis and/or treatment of an individual afflicted with prions comprising at least one agent capable of activating or stimulating at least partially the activity of at least one human cellular protein kinase, phosphatase or cellular signal transduction molecule selected from the group comprising FGF-R1, Tkt, Abl, clk1, MKK7, LIMK-2, CaM-KI, JNK2, CDC2, PRK, PTP-SL, PTP-zeta, HSP86, and GPIR-1.

48. Composition according claim 46 or 47, wherein the agent is at least one compound of the general formula (I) and/or pharmaceutically acceptable salts thereof.


SEQUENCE LISTING

<110> Axxima Pharmaceuticals AG

<120> Human cellular protein kinases and phosphatases as
      targets for dignosis and treatment of prion diseases

<130> AXX-P11001-WO

<140> 0111858.5
<141> 2001-05-16

<150> US 60/293,528
<151> 2001-05-29

<160>

<170> PatentIn Ver. 2.1

<210> 1
<211> 2662
<212> DNA
<213> Homo sapiens

<220> FGF-R1
<223> Description of Sequence: N/A

<400> 1
tcagtttgaa aaggaggatc gagctcactc gtggagtatc catggagatg tggagccttg 60
tcaccaacct ctaactgcag aactgggatg tggagctgga agtgcctcct cttctgggct 120
gtgctggtca cagccacact ctgcaccgct aggccgtccc cgaccttgcc tgaacaagcc 180
cagccctggg gagcccctgt ggaagtggag tccttcctgg tccaccccgg tgacctgctg 240
cagcttcgct gtcggctgcg ggacgatgtg cagagcatca actggctgcg ggacggggtg 300
cagctggcgg aaagcaaccg cacccgcatc acaggggagg aggtggaggt gcaggactcc 360
gtgcccgcag actccggcct ctatgcttgc gtaaccagca gcccctcggg cagtgacacc 420
acctacttct ccgtcaatgt ttcagatgct ctcccctcct cggaggatga tgatgatgat 480
gatgactcct cttcagagga gaaagaaaca gataacacca aaccaaaccg tatgcccgta 540
gctccatatt ggacatcccc agaaaagatg gaaaagaaat tgcatgcagt gccggctgcc 600

aagacagtga agttcaaatg cccttccagt gggaccccaa accccacact gcgctggttg 660
aaaaatggca aagaattcaa acctgaccac agaattggag gctacaaggt ccgttatgcc 720
acctggagca tcataatgga ctctgtggtg ccctctgaca agggcaacta cacctgcatt 780
gtggagaatg agtacggcag catcaaccac acataccagc tggatgtcgt ggagcggtcc 840
cctcaccgcc ccatcctgca agcagggttg cccgccaaca aaacagtggc cctgggtagc 900
aacgtggagt tcatgtgtaa ggtgtacagt gacccgcagc cgcacatcca gtggctaaag 960
cacatcgagg tgaatgggag caagattggc ccagacaacc tgccttatgt ccagatcttg 1020
aagactgctg gagttaatac caccgacaaa gagatggagg tgcttcactt aagaaatgtc 1080
tcctttgagg acgcagggga gtatacgtgc ttggcgggta actctatcgg actctcccat 1140
cactctgcat ggttgaccgt tctggaagcc ctggaagaga ggccggcagt gatgacctcg 1200
cccctgtacc tggagatcat catctattgc acaggggcct tcctcatctc ctgcatggtg 1260
gggtcggtca tcgtctacaa gatgaagagt ggtaccaaga agagtgactt ccacagccag 1320
atggctgtgc acaagctggc caagagcatc cctctgcgca gacaggtaac agtgtctgct 1380
gactccagtg catccatgaa ctctggggtt cttctggttc ggccatcacg gctctcctcc 1440
agtgggactc ccatgctagc aggggtctct gagtatgagc ttcccgaaga ccctcgctgg 1500
gagctgcctc gggacagact ggtcttaggc aaacccctgg gagagggctg ctttgggcag 1560
gtggtgttgg cagaggctat cgggctggac aaggacaaac ccaaccgtgt gaccaaagtg 1620
gctgtgaaga tgttgaagtc ggacgcaaca gagaaagact tgtcagacct gatctcagaa 1680
atggagatga tgaagatgat cgggaagcat aagaatatca tcaacctgct gggggcctgc 1740
acgcaggatg gtcccttgta tgtcatcgtg gagtatgcct ccaagggcaa cctgcgggag 1800
tacctgcagg cccggaggcc cccagggctg gaatactgct acaaccccag ccacaaccca 1860
gaggagcagc tctcctccaa ggacctggtg tcctgcgcct accaggtggc ccgaggcatg 1920
gagtatctgg cctccaagaa gtgcatacac cgagacctgg cagccaggaa tgtcctggtg 1980
acagaggaca atgtgatgaa gatagcagac tttggcctcg cacgggacat tcaccacatc 2040
gactactata aaaagacaac caacggccga ctgcctgtga agtggatggc acccgaggca 2100
ttatttgacc ggatctacac ccaccagagt gatgtgtggt ctttcggggt gctcctgtgg 2160
gagatcttca ctctgggcgg ctccccatac cccggtgtgc ctgtggagga acttttcaag 2220
ctgctgaagg agggtcaccg catggacaag cccagtaact gcaccaacga gctgtacatg 2280
atgatgcggg actgctggca tgcagtgccc tcacagagac ccaccttcaa gcagctggtg 2340
gaagacctgg accgcatcgt ggccttgacc tccaaccagg agtacctgga cctgtccatg 2400
cccctggacc agtactcccc cagctttccc gacacccgga gctctacgtg ctcctcaggg 2460
gaggattccg tcttctctca tgagccgctg cccgaggagc cctgcctgcc ccgacaccca 2520
gcccagcttg ccaatggcgg actcaaacgc cgctgactgc cacccacacg ccctccccag 2580
actccaccgt cagctgtaac cctcacccac agcccctgcc tgggcccacc acctgtccgt 2640
ccctgtcccc tttcctgctg gg 2662

<210> 2

<211> 822

<212> PRT

<213> Homo sapiens

<220>

<223> Description of Sequence: N/A

<400> 2

<210> 3
<211> 3840
<212> DNA
<213> Homo sapiens

<220> Abl
<223> Description of Sequence: N/A

<400> 3
ggccttcccc ctgcgaggat cgccgttggc ccgggttggc tttggaaagc ggcggtggct 60
ttgggccggg ctcggcctcg ggaacgccag gggcccctgg gtgcggacgg gcgcggccag 120
gagggggtta aggcgcaggc ggcggcgggg cgggggcggg cctggcgggc gccctctccg 180
ggccctttgt taacaggcgc gtcccggcca gcggagacgc ggccgccctg ggcgggcgcg 240
ggcggcgggc ggcggtgagg gcggcctgcg ggcggcgccc cgggggccgg gccgagccgg 300
gcctgagccg ggcccggacc gagctgggag aggggctccg gcccgatcgt tcgcttggcg 360
caaaatgttg gagatctgcc tgaagctggt gggctgcaaa tccaagaagg ggctgtcctc 420
gtcctccagc tgttatctgg aagaagccct tcagcggcca gtagcatctg actttgagcc 480
tcagggtctg agtgaagccg ctcgttggaa ctccaaggaa aaccttctcg ctggacccag 540
tgaaaatgac cccaaccttt tcgttgcact gtatgatttt gtggccagtg gagataacac 600
tctaagcata actaaaggtg aaaagctccg ggtcttaggc tataatcaca atggggaatg 660
gtgtgaagcc caaaccaaaa atggccaagg ctgggtccca agcaactaca tcacgccagt 720
caacagtctg gagaaacact cctggtacca tgggcctgtg tcccgcaatg ccgctgagta 780
tccgctgagc agcgggatca atggcagctt cttggtgcgt gagagtgaga gcagtcctag 840
ccagaggtcc atctcgctga gatacgaagg gagggtgtac cattacagga tcaacactgc 900
ttctgatggc aagctctacg tctcctccga gagccgcttc aacaccctgg ccgagttggt 960
tcatcatcat tcaacggtgg ccgacgggct catcaccacg ctccattatc cagccccaaa 1020
gcgcaacaag cccactgtct atggtgtgtc ccccaactac gacaagtggg agatggaacg 1080
cacggacatc accatgaagc acaagctggg cgggggccag tacggggagg tgtacgaggg 1140
cgtgtggaag aaatacagcc tgacggtggc cgtgaagacc ttgaaggagg acaccatgga 1200
ggtggaagag ttcttgaaag aagctgcagt catgaaagag atcaaacacc ctaacctagt 1260
gcagctcctt ggggtctgca cccgggagcc cccgttctat atcatcactg agttcatgac 1320
ctacgggaac ctcctggact acctgaggga gtgcaaccgg caggaggtga acgccgtggt 1380
gctgctgtac atggccactc agatctcgtc agccatggag tacctagaga agaaaaactt 1440
catccacaga gatcttgctg cccgaaactg cctggtaggg gagaaccact tggtgaaggt 1500
agctgatttt ggcctgagca ggttgatgac aggggacacc tacacagccc atgctggagc 1560
caagttcccc atcaaatgga ctgcacccga gagcctggcc tacaacaagt tctccatcaa 1620
gtccgacgtc tgggcatttg gagtattgct ttgggaaatt gctacctatg gcatgtcccc 1680

ttacccggga attgaccgtt cccaggtgta tgagctgcta gagaaggact accgcatgaa 1740
gcgcccagaa ggctgcccag agaaggtcta tgaactcatg cgagcatgtt ggcagtggaa 1800
tccctctgac cggccctcct ttgctgaaat ccaccaagcc tttgaaacaa tgttccagga 1860
atccagtatc tcagacgaag tggaaaagga gctggggaaa caaggcgtcc gtggggctgt 1920
gactaccttg ctgcaggccc cagagctgcc caccaagacg aggacctcca ggagagctgc 1980
agagcacaga gacaccactg acgtgcctga gatgcctcac tccaagggcc agggagagag 2040
cgatcctctg gaccatgagc ctgccgtgtc tccattgctc cctcgaaaag agcgaggtcc 2100
cccggagggc ggcctgaatg aagatgagcg ccttctcccc aaagacaaaa agaccaactt 2160
gttcagcgcc ttgatcaaga agaagaagaa gacagcccca acccctccca aacgcagcag 2220
ctccttccgg gagatggacg gccagccgga gcgcagaggg gccggcgagg aagagggccg 2280
agacatcagc aacggggcac tggctttcac ccccttggac acagctgacc cagccaagtc 2340
cccaaagccc agcaatgggg ctggggtccc caatggagcc ctccgggagt ccgggggctc 2400
aggcttccgg tctccccacc tgtggaagaa gtccagcacg ctgaccagca gccgcctagc 2460
caccggcgag gaggagggcg gtggcagctc cagcaagcgc ttcctgcgct cttgctccgt 2520
ctcctgcgtt ccccatgggg ccaaggacac ggagtggagg tcagtcacgc tgcctcggga 2580
cttgcagtcc acgggaagac agtttgactc gtccacattt ggagggcaca aaagtgagaa 2640
gccggctctg cctcggaaga gggcagggga gaacaggtct gaccaggtga cccgaggcac 2700
agtaacgcct ccccccaggc tggtgaaaaa gaatgaggaa gctgctgatg aggtcttcaa 2760
agacatcatg gagtccagcc cgggctccag cccgcccaac ctgactccaa aacccctccg 2820
gcggcaggtc accgtggccc ctgcctcggg cctcccccac aaggaagaag cctggaaagg 2880
cagtgcctta gggacccctg ctgcagctga gccagtgacc cccaccagca aagcaggctc 2940
aggtgcacca aggggcacca gcaagggccc cgccgaggag tccagagtga ggaggcacaa 3000
gcactcctct gagtcgccag ggagggacaa ggggaaattg tccaagctca aacctgcccc 3060
gccgccccca ccagcagcct ctgcagggaa ggctggagga aagccctcgc agaggcccgg 3120
ccaggaggct gccggggagg cagtcttggg cgcaaagaca aaagccacga gtctggttga 3180
tgctgtgaac agtgacgctg ccaagcccag ccagccggca gagggcctca aaaagcccgt 3240
gctcccggcc actccaaagc cacaccccgc caagccgtcg gggaccccca tcagcccagc 3300
ccccgttccc ctttccacgt tgccatcagc atcctcggcc ttggcagggg accagccgtc 3360
ttccactgcc ttcatccctc tcatatcaac ccgagtgtct cttcggaaaa cccgccagcc 3420
tccagagcgg gccagcggcg ccatcaccaa gggcgtggtc ttggacagca ccgaggcgct 3480
gtgcctcgcc atctctggga actccgagca gatggccagc cacagcgcag tgctggaggc 3540
cggcaaaaac ctctacacgt tctgcgtgag ctatgtggat tccatccagc aaatgaggaa 3600
caagtttgcc ttccgagagg ccatcaacaa actggagaat aatctccggg agcttcagat 3660
ctgcccggcg tcagcaggca gtggtccggc ggccactcag gacttcagca agctcctcag 3720
ttcggtgaag gaaatcagtg acatagtgca gaggtagcag cagtcagggg tcaggtgtca 3780
ggcccgtcgg agctgcctgc agcacatgcg ggctcgccca tacccatgac agtggctgag 3840

<210> 4
<211> 1130
<212> PRT
<213> Homo sapiens

<220>
<223> Description of Sequence: N/A

<400> 4
Met Leu Glu Ile Cys Leu Lys Leu Val Gly Cys Lys Ser Lys Lys Gly
1               5                   10                  15
Leu Ser Ser Ser Ser Ser Cys Tyr Leu Glu Glu Ala Leu Gln Arg Pro
            20                  25                  30
Val Ala Ser Asp Phe Glu Pro Gln Gly Leu Ser Glu Ala Ala Arg Trp
        35                  40                  45
Asn Ser Lys Glu Asn Leu Leu Ala Gly Pro Ser Glu Asn Asp Pro Asn
    50                  55                  60
Leu Phe Val Ala Leu Tyr Asp Phe Val Ala Ser Gly Asp Asn Thr Leu
65                  70                  75                  80
Ser Ile Thr Lys Gly Glu Lys Leu Arg Val Leu Gly Tyr Asn His Asn
                85                  90                  95
Gly Glu Trp Cys Glu Ala Gln Thr Lys Asn Gly Gln Gly Trp Val Pro
            100                 105                 110
Ser Asn Tyr Ile Thr Pro Val Asn Ser Leu Glu Lys His Ser Trp Tyr
        115                 120                 125
His Gly Pro Val Ser Arg Asn Ala Ala Glu Tyr Pro Leu Ser Ser Gly
    130                 135                 140
Ile Asn Gly Ser Phe Leu Val Arg Glu Ser Glu Ser Ser Pro Ser Gln
145                 150                 155                 160
Arg Ser Ile Ser Leu Arg Tyr Glu Gly Arg Val Tyr His Tyr Arg Ile
                165                 170                 175
Asn Thr Ala Ser Asp Gly Lys Leu Tyr Val Ser Ser Glu Ser Arg Phe
            180                 185                 190
Asn Thr Leu Ala Glu Leu Val His His His Ser Thr Val Ala Asp Gly
        195                 200                 205
Leu Ile Thr Thr Leu His Tyr Pro Ala Pro Lys Arg Asn Lys Pro Thr
    210                 215                 220
Val Tyr Gly Val Ser Pro Asn Tyr Asp Lys Trp Glu Met Glu Arg Thr

225                 230                 235                 240
Asp Ile Thr Met Lys His Lys Leu Gly Gly Gly Gln Tyr Gly Glu Val
                245                 250                 255
Tyr Glu Gly Val Trp Lys Lys Tyr Ser Leu Thr Val Ala Val Lys Thr
            260                 265                 270
Leu Lys Glu Asp Thr Met Glu Val Glu Glu Phe Leu Lys Glu Ala Ala
        275                 280                 285
Val Met Lys Glu Ile Lys His Pro Asn Leu Val Gln Leu Leu Gly Val
    290                 295                 300
Cys Thr Arg Glu Pro Pro Phe Tyr Ile Ile Thr Glu Phe Met Thr Tyr
305                 310                 315                 320
Gly Asn Leu Leu Asp Tyr Leu Arg Glu Cys Asn Arg Gln Glu Val Asn
                325                 330                 335
Ala Val Val Leu Leu Tyr Met Ala Thr Gln Ile Ser Ser Ala Met Glu
            340                 345                 350
Tyr Leu Glu Lys Lys Asn Phe Ile His Arg Asp Leu Ala Ala Arg Asn
        355                 360                 365
Cys Leu Val Gly Glu Asn His Leu Val Lys Val Ala Asp Phe Gly Leu
    370                 375                 380
Ser Arg Leu Met Thr Gly Asp Thr Tyr Thr Ala His Ala Gly Ala Lys
385                 390                 395                 400
Phe Pro Ile Lys Trp Thr Ala Pro Glu Ser Leu Ala Tyr Asn Lys Phe
                405                 410                 415
Ser Ile Lys Ser Asp Val Trp Ala Phe Gly Val Leu Leu Trp Glu Ile
            420                 425                 430
Ala Thr Tyr Gly Met Ser Pro Tyr Pro Gly Ile Asp Arg Ser Gln Val
        435                 440                 445
Tyr Glu Leu Leu Glu Lys Asp Tyr Arg Met Lys Arg Pro Glu Gly Cys
    450                 455                 460
Pro Glu Lys Val Tyr Glu Leu Met Arg Ala Cys Trp Gln Trp Asn Pro
465                 470                 475                 480
Ser Asp Arg Pro Ser Phe Ala Glu Ile His Gln Ala Phe Glu Thr Met
                485                 490                 495
Phe Gln Glu Ser Ser Ile Ser Asp Glu Val Glu Lys Glu Leu Gly Lys
            500                 505                 510
Gln Gly Val Arg Gly Ala Val Thr Thr Leu Leu Gln Ala Pro Glu Leu
        515                 520                 525
Pro Thr Lys Thr Arg Thr Ser Arg Arg Ala Ala Glu His Arg Asp Thr

    530                 535                 540
Thr Asp Val Pro Glu Met Pro His Ser Lys Gly Gln Gly Glu Ser Asp
545                 550                 555                 560
Pro Leu Asp His Glu Pro Ala Val Ser Pro Leu Leu Pro Arg Lys Glu
                565                 570                 575
Arg Gly Pro Pro Glu Gly Gly Leu Asn Glu Asp Glu Arg Leu Leu Pro
            580                 585                 590
Lys Asp Lys Lys Thr Asn Leu Phe Ser Ala Leu Ile Lys Lys Lys Lys
        595                 600                 605
Lys Thr Ala Pro Thr Pro Pro Lys Arg Ser Ser Ser Phe Arg Glu Met
    610                 615                 620
Asp Gly Gln Pro Glu Arg Arg Gly Ala Gly Glu Glu Glu Gly Arg Asp
625                 630                 635                 640
Ile Ser Asn Gly Ala Leu Ala Phe Thr Pro Leu Asp Thr Ala Asp Pro
                645                 650                 655
Ala Lys Ser Pro Lys Pro Ser Asn Gly Ala Gly Val Pro Asn Gly Ala
            660                 665                 670
Leu Arg Glu Ser Gly Gly Ser Gly Phe Arg Ser Pro His Leu Trp Lys
        675                 680                 685
Lys Ser Ser Thr Leu Thr Ser Ser Arg Leu Ala Thr Gly Glu Glu Glu
    690                 695                 700
Gly Gly Gly Ser Ser Ser Lys Arg Phe Leu Arg Ser Cys Ser Val Ser
705                 710                 715                 720
Cys Val Pro His Gly Ala Lys Asp Thr Glu Trp Arg Ser Val Thr Leu
                725                 730                 735
Pro Arg Asp Leu Gln Ser Thr Gly Arg Gln Phe Asp Ser Ser Thr Phe
            740                 745                 750
Gly Gly His Lys Ser Glu Lys Pro Ala Leu Pro Arg Lys Arg Ala Gly
        755                 760                 765
Glu Asn Arg Ser Asp Gln Val Thr Arg Gly Thr Val Thr Pro Pro Pro
    770                 775                 780
Arg Leu Val Lys Lys Asn Glu Glu Ala Ala Asp Glu Val Phe Lys Asp
785                 790                 795                 800
Ile Met Glu Ser Ser Pro Gly Ser Ser Pro Pro Asn Leu Thr Pro Lys
                805                 810                 815
Pro Leu Arg Arg Gln Val Thr Val Ala Pro Ala Ser Gly Leu Pro His
            820                 825                 830
Lys Glu Glu Ala Trp Lys Gly Ser Ala Leu Gly Thr Pro Ala Ala Ala

        835                 840                 845
Glu Pro Val Thr Pro Thr Ser Lys Ala Gly Ser Gly Ala Pro Arg Gly
    850                 855                 860
Thr Ser Lys Gly Pro Ala Glu Glu Ser Arg Val Arg Arg His Lys His
865                 870                 875                 880
Ser Ser Glu Ser Pro Gly Arg Asp Lys Gly Lys Leu Ser Lys Leu Lys
                885                 890                 895
Pro Ala Pro Pro Pro Pro Pro Ala Ala Ser Ala Gly Lys Ala Gly Gly
            900                 905                 910
Lys Pro Ser Gln Arg Pro Gly Gln Glu Ala Ala Gly Glu Ala Val Leu
        915                 920                 925
Gly Ala Lys Thr Lys Ala Thr Ser Leu Val Asp Ala Val Asn Ser Asp
    930                 935                 940
Ala Ala Lys Pro Ser Gln Pro Ala Glu Gly Leu Lys Lys Pro Val Leu
945                 950                 955                 960
Pro Ala Thr Pro Lys Pro His Pro Ala Lys Pro Ser Gly Thr Pro Ile
                965                 970                 975
Ser Pro Ala Pro Val Pro Leu Ser Thr Leu Pro Ser Ala Ser Ser Ala
            980                 985                 990
Leu Ala Gly Asp Gln Pro Ser Ser Thr Ala Phe Ile Pro Leu Ile Ser
        995                 1000                1005
Thr Arg Val Ser Leu Arg Lys Thr Arg Gln Pro Pro Glu Arg Ala Ser
    1010                1015                1020
Gly Ala Ile Thr Lys Gly Val Val Leu Asp Ser Thr Glu Ala Leu Cys
1025                1030                1035                1040
Leu Ala Ile Ser Gly Asn Ser Glu Gln Met Ala Ser His Ser Ala Val
                1045                1050                1055
Leu Glu Ala Gly Lys Asn Leu Tyr Thr Phe Cys Val Ser Tyr Val Asp
            1060                1065                1070
Ser Ile Gln Gln Met Arg Asn Lys Phe Ala Phe Arg Glu Ala Ile Asn
        1075                1080                1085
Lys Leu Glu Asn Asn Leu Arg Glu Leu Gln Ile Cys Pro Ala Ser Ala
    1090                1095                1100
Gly Ser Gly Pro Ala Ala Thr Gln Asp Phe Ser Lys Leu Leu Ser Ser
1105                1110                1115                1120
Val Lys Glu Ile Ser Asp Ile Val Gln Arg
                1125                1130

<210> 5

<211> 1461

<212> DNA

<213> Homo sapiens

<220> MKK7

<223> Description of Sequence: N/A

<400> 5

aggcggtgtt tgtctgccgg actgacgggc ggccgggcgg tgcgcggcgg cggtggcggc 60

ggggaaaatg gcggcgtcct ccctggaaca gaagctgtcc cgcctggaag caaagctgaa 120

gcaggagaac cgggaggccc ggcggaggat cgacctcaac ctggatatca gcccccagcg 180

gcccaggccc accctgcagc tcccgctggc caacgatggg ggcagccgct cgccatcctc 240

agagagctcc ccgcagcacc ccacgccccc cgcccggccc cgccacatgc tggggctccc 300

gtcaaccctg ttcacacccc gcagcatgga gagcattgag attgaccaga agctgcagga 360

gatcatgaag cagacgggct acctgaccat cgggggccag cgctaccagg cagaaatcaa 420

cgacctggag aacttgggcg agatgggcag cggcacctgc ggccaggtgt ggaagatgcg 480

cttccggaag accggccacg tcattgccgt taagcaaatg cggcgctccg ggaacaagga 540

ggagaacaag cgcatcctca tggacctgga tgtggtgctg aagagccacg actgccccta 600

catcgtgcag tgctttggga cgttcatcac caacacggac gtcttcatcg ccatggagct 660

catgggcacc tgcgctgaga agctcaagaa gcggatgcag ggccccatcc ccgagcgcat 720

tctgggcaag atgacagtgg cgattgtgaa ggcgctgtac tacctgaagg agaagcacgg 780

tgtcatccac cgcgacgtca agccctccaa catcctgctg gacgagcggg gccagatcaa 840

gttctgcgac ttcggcatca gcggccgcct ggtggactcc aaagccaaga cgcggagcgc 900

cggctgtgcc gcctacatgg cacccgagcg cattgacccc ccagacccca ccaagccgga 960

ctatgacatc cgggccgacg tatggagcct gggcatctcg ctggtggagc tggcaacagg 1020

acagtttccc tacaagaact gcaagacgga ctttgaggtc ctcaccaaag tcctacagga 1080

agagcccccg cttctgcccg gacacatggg cttctcgggg gacttccagt ccttcgtcaa 1140

agactgcctt actaaagatc acaggaagag accaaagtat aataagctac ttgaacacag 1200

cttcatcaag cgctacgaga cgctggaggt ggacgtggcg tcctggttca aggatgtcat 1260

ggcgaagact gagtcaccgc ggactagcgg cgtcctgagc cagccccacc tgcccttctt 1320

caggtagctg cttggcggcg gccagcccca cagggggcca ggggcatggc cacaggcccc 1380

cctccccact tggccaccca gctgcctgcc aggggagacc tgggacctgg acggccacct 1440

aggactgagg acagagagtg g 1461

<210> 6
<211> 419
<212> PRT
<213> Homo sapiens

<220>
<223> Description of Sequence: N/A

<400> 6

<210> 7
<211> 1050
<212> DNA
<213> Homo sapiens

<220> CDC2
<223> Description of Sequence: N/A

<400> 7
gggggggggg ggcacttggc ttcaaagctg gctcttggaa attgagcgga gacgagcggc 60
ttgttgtagc tgccgtgcgg ccgccgcgga ataataagcc gggatctacc ataccattga 120

ctaactatgg aagattatac caaaatagag aaaattggag aaggtaccta tggagttgtg 180
tataagggta gacacaaaac tacaggtcaa gtggtagcca tgaaaaaaat cagactagaa 240
agtgaagagg aaggggttcc tagtactgca attcgggaaa tttctctatt aaaggaactt 300
cgtcatccaa atatagtcag tcttcaggat gtgcttatgc aggattccag gttatatctc 360
atctttgagt ttctttccat ggatctgaag aaatacttgg attctatccc tcctggtcag 420
tacatggatt cttcacttgt taagagttat ttataccaaa tcctacaggg gattgtgttt 480
tgtcactcta gaagagttct tcacagagac ttaaaacctc aaaatctctt gattgatgac 540
aaaggaacaa ttaaactggc tgattttggc cttgccagag cttttggaat acctatcaga 600
gtatatacac atgaggtagt aacactctgg tacagatctc cagaagtatt gctggggtca 660
gctcgttact caactccagt tgacatttgg agtataggca ccatatttgc tgaactagca 720
actaagaaac cacttttcca tggggattca gaaattgatc aactcttcag gattttcaga 780
gctttgggca ctcccaataa tgaagtgtgg ccagaagtgg aatctttaca ggactataag 840
aatacatttc ccaaatggaa accaggaagc ctagcatccc atgtcaaaaa cttggatgaa 900
aatggcttgg atttgctctc gaaaatgtta atctatgatc cagccaaacg aatttctggc 960
aaaatggcac tgaatcatcc atattttaat gatttggaca atcagattaa gaagatgtag 1020
ctttctgaca aaaagtttcc atatgttatg 1050

<210> 8
<211> 297
<212> PRT
<213> Homo sapiens

<220>
<223> Description of Sequence: N/A

<400> 8
Met Glu Asp Tyr Thr Lys Ile Glu Lys Ile Gly Glu Gly Thr Tyr Gly
1               5                   10                  15
Val Val Tyr Lys Gly Arg His Lys Thr Thr Gly Gln Val Val Ala Met
            20                  25                  30
Lys Lys Ile Arg Leu Glu Ser Glu Glu Glu Gly Val Pro Ser Thr Ala
        35                  40                  45
Ile Arg Glu Ile Ser Leu Leu Lys Glu Leu Arg His Pro Asn Ile Val
    50                  55                  60
Ser Leu Gln Asp Val Leu Met Gln Asp Ser Arg Leu Tyr Leu Ile Phe
65                  70                  75                  80
Glu Phe Leu Ser Met Asp Leu Lys Lys Tyr Leu Asp Ser Ile Pro Pro

                        85                  90                  95
Gly Gln Tyr Met Asp Ser Ser Leu Val Lys Ser Tyr Leu Tyr Gln Ile
                100                 105                 110
Leu Gln Gly Ile Val Phe Cys His Ser Arg Arg Val Leu His Arg Asp
            115                 120                 125
Leu Lys Pro Gln Asn Leu Leu Ile Asp Asp Lys Gly Thr Ile Lys Leu
    130                 135                 140
Ala Asp Phe Gly Leu Ala Arg Ala Phe Gly Ile Pro Ile Arg Val Tyr
145                 150                 155                 160
Thr His Glu Val Val Thr Leu Trp Tyr Arg Ser Pro Glu Val Leu Leu
                165                 170                 175
Gly Ser Ala Arg Tyr Ser Thr Pro Val Asp Ile Trp Ser Ile Gly Thr
                180                 185                 190
Ile Phe Ala Glu Leu Ala Thr Lys Lys Pro Leu Phe His Gly Asp Ser
            195                 200                 205
Glu Ile Asp Gln Leu Phe Arg Ile Phe Arg Ala Leu Gly Thr Pro Asn
    210                 215                 220
Asn Glu Val Trp Pro Glu Val Glu Ser Leu Gln Asp Tyr Lys Asn Thr
225                 230                 235                 240
Phe Pro Lys Trp Lys Pro Gly Ser Leu Ala Ser His Val Lys Asn Leu
                245                 250                 255
Asp Glu Asn Gly Leu Asp Leu Leu Ser Lys Met Leu Ile Tyr Asp Pro
                260                 265                 270
Ala Lys Arg Ile Ser Gly Lys Met Ala Leu Asn His Pro Tyr Phe Asn
            275                 280                 285
Asp Leu Asp Asn Gln Ile Lys Lys Met
    290                 295

<210> 9
<211> 1480
<212> DNA
<213> Homo sapiens

<220> CamKI
<223> Description of Sequence: N/A

<400> 9
gaattccgag caagagcgcg ggcgggtggc ccaggcacgc agcgggtgag gaccgcgccc 60
acagctcggc gccaaccacc gcgggcctcc cagccagccc cgcnnngagc cgcaggancc 120
ctggctgtgg tcggggggca gtgggccatg ctgggggcag tggaaggccc caggtggaag 180
caggcggagg acattagaga catctacgac ttccgagatg ttctgggcac gggggccttc 240
tcggaggtga tcctggcaga agataagagg acgcagaagc tggtggccat caaatgcatt 300
gccaaggagg ccctggaggg caaggaaggc agcatggaga atgagattgc tgtcctgcac 360
aagatcaagc accccaacat tgtagccctg gatgacatct atgagagtgg gggccacctc 420
tacctcatca tgcagctggt gtcgggtggg gagctctttg accgtattgt ggaaaaaggc 480
ttctacacgg agcgggacgc cagccgcctc atcttccagg tgctggatgc tgtgaaatac 540
ctgcatgacc tgggcattgt acaccgggat ctcaagccag agaatctgct gtactacagc 600
ctggatgaag actccaaaat catgatctcc gactttggcc tctccaagat ggaggacccg 660
ggcagtgtgc tctccaccgc ctgtggaact ccgggatacg tggcccctga agtcctggcc 720
cagaagccct acagcaaggc tgtggattgc tggtccatag gtgtcatcgc ctacatcttg 780
ctctgcggtt accctccctt ctatgacgag aatgatgcca aactctttga acagattttg 840
aaggccgagt acgagtttga ctctccttac tgggacgaca tctctgactc tgccaaagat 900
ttcatccggc acttgatgga gaaggaccca gagaaaagat tcacctgtga gcaggccttg 960
cagcacccat ggattgcagg agatacagct ctagataaga atatccacca gtcggtgagt 1020
gagcagatca agaagaactt tgccaagagc aagtggaagc aagccttcaa tgccacggct 1080
gtggtgcggc acatgaggaa actgcagctg ggcaccagcc aggaggggca ggggcagacg 1140
gcgagccatg gggagctgct gacaccagtg gctggggggc cggcagctgg ctgttgctgt 1200
cgagactgct gcgtggagcc gggcacagaa ctgtccccca cactgcccca ccagctctag 1260
ggccctggac ctcgggtcat gatcctctgc gtgggagggc ttgggggcca gcctgctccc 1320
cttccctccc tgaaccggga gtttctctgc cctgtcccct cctcacctgc ttccctacca 1380
ctcctcactg cattttccat acaaatgttt ctattttatt gttccttctt gtaataaagg 1440
gaagataaaa ccaaaaaaaa aaaaaaaaaa acggaattcc 1480

<210> 10
<211> 370
<212> PRT
<213> Homo sapiens

<220>
<223> Description of Sequence: N/A

<400> 10
Met Leu Gly Ala Val Glu Gly Pro Arg Trp Lys Gln Ala Glu Asp Ile

1               5                   10                  15
Arg Asp Ile Tyr Asp Phe Arg Asp Val Leu Gly Thr Gly Ala Phe Ser
            20                  25                  30
Glu Val Ile Leu Ala Glu Asp Lys Arg Thr Gln Lys Leu Val Ala Ile
        35                  40                  45
Lys Cys Ile Ala Lys Glu Ala Leu Glu Gly Lys Glu Gly Ser Met Glu
    50                  55                  60
Asn Glu Ile Ala Val Leu His Lys Ile Lys His Pro Asn Ile Val Ala
65                  70                  75                  80
Leu Asp Asp Ile Tyr Glu Ser Gly Gly His Leu Tyr Leu Ile Met Gln
                85                  90                  95
Leu Val Ser Gly Gly Glu Leu Phe Asp Arg Ile Val Glu Lys Gly Phe
            100                 105                 110
Tyr Thr Glu Arg Asp Ala Ser Arg Leu Ile Phe Gln Val Leu Asp Ala
        115                 120                 125
Val Lys Tyr Leu His Asp Leu Gly Ile Val His Arg Asp Leu Lys Pro
    130                 135                 140
Glu Asn Leu Leu Tyr Tyr Ser Leu Asp Glu Asp Ser Lys Ile Met Ile
145                 150                 155                 160
Ser Asp Phe Gly Leu Ser Lys Met Glu Asp Pro Gly Ser Val Leu Ser
                165                 170                 175
Thr Ala Cys Gly Thr Pro Gly Tyr Val Ala Pro Glu Val Leu Ala Gln
            180                 185                 190
Lys Pro Tyr Ser Lys Ala Val Asp Cys Trp Ser Ile Gly Val Ile Ala
        195                 200                 205
Tyr Ile Leu Leu Cys Gly Tyr Pro Pro Phe Tyr Asp Glu Asn Asp Ala
    210                 215                 220
Lys Leu Phe Glu Gln Ile Leu Lys Ala Glu Tyr Glu Phe Asp Ser Pro
225                 230                 235                 240
Tyr Trp Asp Asp Ile Ser Asp Ser Ala Lys Asp Phe Ile Arg His Leu
                245                 250                 255
Met Glu Lys Asp Pro Glu Lys Arg Phe Thr Cys Glu Gln Ala Leu Gln
            260                 265                 270
His Pro Trp Ile Ala Gly Asp Thr Ala Leu Asp Lys Asn Ile His Gln
        275                 280                 285
Ser Val Ser Glu Gln Ile Lys Lys Asn Phe Ala Lys Ser Lys Trp Lys
    290                 295                 300
Gln Ala Phe Asn Ala Thr Ala Val Val Arg His Met Arg Lys Leu Gln

305                 310                 315                 320
Leu Gly Thr Ser Gln Glu Gly Gln Gly Gln Thr Ala Ser His Gly Glu
                325                 330                 335
Leu Leu Thr Pro Val Ala Gly Gly Pro Ala Ala Gly Cys Cys Cys Arg
            340                 345                 350
Asp Cys Cys Val Glu Pro Gly Thr Glu Leu Ser Pro Thr Leu Pro His
        355                 360                 365
Gln Leu
    370

<210> 11
<211> 1782
<212> DNA
<213> Homo sapiens

<220> JNK2
<223> Description of Sequence: N/A

<400> 11
gggcgggcga gggatctgaa acttgcccac ccttcgggat attgcaggac gctgcatcat 60
gagcgacagt aaatgtgaca gtcagtttta tagtgtgcaa gtggcagact caaccttcac 120
tgtcctaaaa cgttaccagc agctgaaacc aattggctct ggggcccaag ggattgtttg 180
tgctgcattt gatacagttc ttgggataag tgttgcagtc aagaaactaa gccgtccttt 240
tcagaaccaa actcatgcaa agagagctta tcgtgaactt gtcctcttaa aatgtgtcaa 300
tcataaaaat ataattagtt tgttaaatgt gtttacacca caaaaaactc tagaagaatt 360
tcaagatgtg tatttggtta tggaattaat ggatgctaac ttatgtcagg ttattcacat 420
ggagctggat catgaaagaa tgtcctacct tctttaccag atgctttgtg gtattaaaca 480
tctgcattca gctggtataa ttcatagaga tttgaagcct agcaacattg ttgtgaaatc 540
agactgcacc ctgaagatcc ttgactttgg cctggcccgg acagcgtgca ctaacttcat 600
gatgacccct tacgtggtga cacggtacta ccgggcgccc gaagtcatcc tgggtatggg 660
ctacaaagag aacgttgata tctggtcagt gggttgcatc atgggagagc tggtgaaagg 720
ttgtgtgata ttccaaggca ctgaccatat tgatcagtgg aataaagtta ttgagcagct 780
gggaacacca tcagcagagt tcatgaagaa acttcagcca actgtgagga attatgtcga 840
aaacagacca aagtatcctg gaatcaaatt tgaagaactc tttccagatt ggatattccc 900
atcagaatct gagcgagaca aaataaaaac aagtcaagcc agagatctgt tatcaaaaat 960
gttagtgatt gatcctgaca agcggatctc tgtagacgaa gctctgcgtc acccatacat 1020

cactgtttgg tatgaccccg ccgaagcaga agccccacca cctcaaattt atgatgccca 1080
gttggaagaa agagaacatg caattgaaga atggaaagag ctaatttaca aagaagtcat 1140
ggattgggaa gaaagaagca agaatggtgt tgtaaaagat cagccttcag atgcagcagt 1200
aagtagcaac gccactcctt ctcagtcttc atcgatcaat gacatttcat ccatgtccac 1260
tgagcagacg ctggcctcag acacagacag cagtcttgat gcctcgacgg gaccccttga 1320
aggctgtcga tgataggtta gaaatagcaa acctgtcagc attgaaggaa ctctcacctc 1380
cgtgggcctg aaatgcttgg gagttgatgg aaccaaatag aaaaactcca tgttctgcat 1440
gtaagaaaca caatgccttg ccctattcag acctgatagg attgcctgct tagatgataa 1500
aatgaggcag aatatgtctg aagaaaaaaa ttgcaagcca cacttctaga gattttgttc 1560
aagatcattt caggtgagca gttagagtag gtgaatttgt ttcaaattgt actagtgaca 1620
gtttctcatc atctgtaact gttgagatgt atgtgcatgt gaccacaaat gcttgcttgg 1680
acttgcccat ctagcacttt ggaaatcagt atttaaatgc caaataatct tccaggtagt 1740
gctgcttctg aagttatctc ttaatcctct taagtaattt gg 1782

<210> 12
<211> 424
<212> PRT
<213> Homo sapiens

<220>
<223> Description of Sequence: N/A

<400> 12

<210> 13
<211> 3668
<212> DNA
<213> Homo sapiens

<220> LIMK-2
<223> Description of Sequence: N/A

<400> 13
gtggtcttcc cgcgcctgag gcggcggcgg caggagctga ggggagttgt agggaactga 60
ggggagctgc tgtgtccccc gcctcctcct ccccatttcc gggctcccgg gaccatgtcc 120
gcgctggcgg gtgaagatgt ctggaggtgt ccaggctgtg gggaccacat tgctccaagc 180
cagatatggt acaggactgt caacgaaacc tggcacggct cttgcttccg gtgttcagaa 240
tgccaggatt ccctcaccaa ctggtactat gagaaggatg ggaagctcta ctgccccaag 300
gactactggg ggaagtttgg ggagttctgt catgggtgct ccctgctgat gacagggcct 360
tttatggtgg ctggggagtt caagtaccac ccagagtgct ttgcctgtat gagctgcaag 420
gtgatcattg aggatgggga tgcatatgca ctggtgcagc atgccaccct ctactgtggg 480
aagtgccaca atgaggtggt gctggcaccc atgtttgaga gactctccac agagtctgtt 540
caggagcagc tgccctactc tgtcacgctc atctccatgc cggccaccac tgaaggcagg 600
cggggcttct ccgtgtccgt ggagagtgcc tgctccaact acgccaccac tgtgcaagtg 660
aaagaggtca accggatgca catcagtccc aacaatcgaa acgccatcca ccctggggac 720
cgcatcctgg agatcaatgg gacccccgtc cgcacacttc gagtggagga ggtggaggat 780
gcaattagcc agacgagcca gacacttcag ctgttgattg aacatgaccc cgtctcccaa 840
cgcctggacc agctgcggct ggaggcccgg ctcgctcctc acatgcagaa tgccggacac 900
ccccacgccc tcagcaccct ggacaccaag gagaatctgg aggggacact gaggagacgt 960
tccctaaggc gcagtaacag tatctccaag tcccctggcc ccagctcccc aaaggagccc 1020
ctgctgttca gccgtgacat cagccgctca gaatcccttc gttgttccag cagctattca 1080
cagcagatct tccggccctg tgacctaatc catggggagg tcctggggaa gggcttcttt 1140
gggcaggcta tcaaggtgac acacaaagcc acgggcaaag tgatggtcat gaaagagtta 1200
attcgatgtg atgaggagac ccagaaaact tttctgactg aggtgaaagt gatgcgcagc 1260
ctggaccacc ccaatgtgct caagttcatt ggtgtgctgt acaaggataa gaagctgaac 1320
ctgctgacag agtacattga ggggggcaca ctgaaggact ttctgcgcag tatggatccg 1380
ttcccctggc agcagaaggt caggtttgcc aaaggaatcg cctccggaat ggcctatttg 1440

cactctatgt gcatcatcca ccgggatctg aactcgcaca actgcctcat caagttggac 1500
aagactgtgg tggtggcaga ctttgggctg tcacggctca tagtggaaga gaggaaaagg 1560
gcccccatgg agaaggccac caccaagaaa cgcaccttgc gcaagaacga ccgcaagaag 1620
cgctacacgg tggtgggaaa cccctactgg atggcccctg agatgctgaa cggaaagagc 1680
tatgatgaga cggtggatat cttctccttt gggatcgttc tctgtgagat cattgggcag 1740
gtgtatgcag atcctgactg ccttccccga acactggact ttggcctcaa cgtgaagctt 1800
ttctgggaga agtttgttcc cacagattgt cccccggcct tcttcccgct ggccgccatc 1860
tgctgcagac tggagcctga gagcagacca gcattctcga aattggagga ctcctttgag 1920
gccctctccc tgtacctggg ggagctgggc atcccgctgc ctgcagagct ggaggagttg 1980
gaccacactg tgagcatgca gtacggcctg acccgggact cacctcccta gccctggccc 2040
agccccctgc aggggggtgt tctacagcca gcattgcccc tctgtgcccc attcctgctg 2100
tgagcagggc cgtccgggct tcctgtggat tggcggaatg tttagaagca gaacaagcca 2160
ttcctattac ctccccagga ggcaagtggg cgcagcacca gggaaatgta tctccacagg 2220
ttctggggcc tagttactgt ctgtaaatcc aatacttgcc tgaaagctgt gaagaagaaa 2280
aaaacccctg gcctttgggc caggaggaat ctgttactcg aatccaccca ggaactccct 2340
ggcagtggat tgtgggaggc tcttgcttac actaatcagc gtgacctgga cctgctgggc 2400
aggatcccag ggtgaacctg cctgtgaact ctgaagtcac tagtccagct gggtgcagga 2460
ggacttcaag tgtgtggacg aaagaaagac tgatggctca aagggtgtga aaaagtcagt 2520
gatgctcccc ctttctactc cagatcctgt ccttcctgga gcaaggttga gggagtaggt 2580
tttgaagagt cccttaatat gtggtggaac aggccaggag ttagagaaag ggctggcttc 2640
tgtttacctg ctcactggct ctagccagcc cagggaccac atcaatgtga gaggaagcct 2700
ccacctcatg ttttcaaact taatactgga gactggctga gaacttacgg acaacatcct 2760
ttctgtctga aacaaacagt cacaagcaca ggaagaggct gggggactag aaagaggccc 2820
tgccctctag aaagctcaga tcttggcttc tgttactcat actcgggtgg gctccttagt 2880
cagatgccta aaacattttg cctaaagctc gatgggttct ggaggacagt gtggcttgtc 2940
acaggcctag agtctgaggg aggggagtgg gagtctcagc aatctcttgg tcttggcttc 3000
atggcaacca ctgctcaccc ttcaacatgc ctggtttagg cagcagcttg ggctgggaag 3060
aggtggtggc agagtctcaa agctgagatg ctgagagaga tagctccctg agctgggcca 3120
tctgacttct acctcccatg tttgctctcc caactcatta gctcctgggc agcatcctcc 3180
tgagccacat gtgcaggtac tggaaaacct ccatcttggc tcccagagct ctaggaactc 3240
ttcatcacaa ctagatttgc ctcttctaag tgtctatgag cttgcaccat atttaataaa 3300
ttgggaatgg gtttggggta ttaatgcaat gtgtggtggt tgtattggag cagggggaat 3360
tgataaagga gagtggttgc tgttaatatt atcttatcta ttgggtggta tgtgaaatat 3420
tgtacataga cctgatgagt tgtgggacca gatgtcatct ctggtcagag tttacttgct 3480
atatagactg tacttatgtg tgaagtttgc aagcttgctt tagggctgag ccctggactc 3540
ccagcagcag cacagttcag cattgtgtgg ctggttgttt cctggctgtc cccagcaagt 3600
gtaggagtgg tgggcctgaa ctgggccatt gatcagacta aataaattaa gcagttaaca 3660
taactggc 3668

<210> 14

<211> 638

<212> PRT

<213> Homo sapiens

<220>

<223> Description of Sequence: N/A

<400> 14

Met Ser Ala Leu Ala Gly Glu Asp Val Trp Arg Cys Pro Gly Cys Gly
1               5                   10                  15

Asp His Ile Ala Pro Ser Gln Ile Trp Tyr Arg Thr Val Asn Glu Thr
            20                  25                  30

Trp His Gly Ser Cys Phe Arg Cys Ser Glu Cys Gln Asp Ser Leu Thr
        35                  40                  45

Asn Trp Tyr Tyr Glu Lys Asp Gly Lys Leu Tyr Cys Pro Lys Asp Tyr
    50                  55                  60

Trp Gly Lys Phe Gly Glu Phe Cys His Gly Cys Ser Leu Leu Met Thr
65                  70                  75                  80

Gly Pro Phe Met Val Ala Gly Glu Phe Lys Tyr His Pro Glu Cys Phe
                85                  90                  95

Ala Cys Met Ser Cys Lys Val Ile Ile Glu Asp Gly Asp Ala Tyr Ala
            100                 105                 110

Leu Val Gln His Ala Thr Leu Tyr Cys Gly Lys Cys His Asn Glu Val
        115                 120                 125

Val Leu Ala Pro Met Phe Glu Arg Leu Ser Thr Glu Ser Val Gln Glu
    130                 135                 140

Gln Leu Pro Tyr Ser Val Thr Leu Ile Ser Met Pro Ala Thr Thr Glu
145                 150                 155                 160

Gly Arg Arg Gly Phe Ser Val Ser Val Glu Ser Ala Cys Ser Asn Tyr
                165                 170                 175

Ala Thr Thr Val Gln Val Lys Glu Val Asn Arg Met His Ile Ser Pro
            180                 185                 190

Asn Asn Arg Asn Ala Ile His Pro Gly Asp Arg Ile Leu Glu Ile Asn
        195                 200                 205

Gly Thr Pro Val Arg Thr Leu Arg Val Glu Glu Val Glu Asp Ala Ile

210                 215                 220
Ser Gln Thr Ser Gln Thr Leu Gln Leu Leu Ile Glu His Asp Pro Val
225                 230                 235                 240
Ser Gln Arg Leu Asp Gln Leu Arg Leu Glu Ala Arg Leu Ala Pro His
                245                 250                 255
Met Gln Asn Ala Gly His Pro His Ala Leu Ser Thr Leu Asp Thr Lys
            260                 265                 270
Glu Asn Leu Glu Gly Thr Leu Arg Arg Arg Ser Leu Arg Arg Ser Asn
        275                 280                 285
Ser Ile Ser Lys Ser Pro Gly Pro Ser Ser Pro Lys Glu Pro Leu Leu
    290                 295                 300
Phe Ser Arg Asp Ile Ser Arg Ser Glu Ser Leu Arg Cys Ser Ser Ser
305                 310                 315                 320
Tyr Ser Gln Gln Ile Phe Arg Pro Cys Asp Leu Ile His Gly Glu Val
                325                 330                 335
Leu Gly Lys Gly Phe Phe Gly Gln Ala Ile Lys Val Thr His Lys Ala
            340                 345                 350
Thr Gly Lys Val Met Val Met Lys Glu Leu Ile Arg Cys Asp Glu Glu
        355                 360                 365
Thr Gln Lys Thr Phe Leu Thr Glu Val Lys Val Met Arg Ser Leu Asp
    370                 375                 380
His Pro Asn Val Leu Lys Phe Ile Gly Val Leu Tyr Lys Asp Lys Lys
385                 390                 395                 400
Leu Asn Leu Leu Thr Glu Tyr Ile Glu Gly Gly Thr Leu Lys Asp Phe
                405                 410                 415
Leu Arg Ser Met Asp Pro Phe Pro Trp Gln Gln Lys Val Arg Phe Ala
            420                 425                 430
Lys Gly Ile Ala Ser Gly Met Ala Tyr Leu His Ser Met Cys Ile Ile
        435                 440                 445
His Arg Asp Leu Asn Ser His Asn Cys Leu Ile Lys Leu Asp Lys Thr
    450                 455                 460
Val Val Val Ala Asp Phe Gly Leu Ser Arg Leu Ile Val Glu Glu Arg
465                 470                 475                 480
Lys Arg Ala Pro Met Glu Lys Ala Thr Thr Lys Lys Arg Thr Leu Arg
                485                 490                 495
Lys Asn Asp Arg Lys Lys Arg Tyr Thr Val Val Gly Asn Pro Tyr Trp
            500                 505                 510
Met Ala Pro Glu Met Leu Asn Gly Lys Ser Tyr Asp Glu Thr Val Asp

        515                 520                 525
Ile Phe Ser Phe Gly Ile Val Leu Cys Glu Ile Ile Gly Gln Val Tyr
    530                 535                 540
Ala Asp Pro Asp Cys Leu Pro Arg Thr Leu Asp Phe Gly Leu Asn Val
545                 550                 555                 560
Lys Leu Phe Trp Glu Lys Phe Val Pro Thr Asp Cys Pro Pro Ala Phe
                565                 570                 575
Phe Pro Leu Ala Ala Ile Cys Cys Arg Leu Glu Pro Glu Ser Arg Pro
            580                 585                 590
Ala Phe Ser Lys Leu Glu Asp Ser Phe Glu Ala Leu Ser Leu Tyr Leu
        595                 600                 605
Gly Glu Leu Gly Ile Pro Leu Pro Ala Glu Leu Glu Glu Leu Asp His
    610                 615                 620
Thr Val Ser Met Gln Tyr Gly Leu Thr Arg Asp Ser Pro Pro
625                 630                 635

<210> 15
<211> 2169
<212> DNA
<213> Homo sapiens

<220> PRK
<223> Description of Sequence: N/A

<400> 15
ccgcctccga gtgccttgcg cggacctgag ctggagatgc tggccgggct accgacgtca 60
gaccccgggc gcctcatcac ggacccgcgc agcggccgca cctacctcaa aggccgcttg 120
ttgggcaagg ggggcttcgc ccgctgctac gaggccactg acacagagac tggcagcgcc 180
tacgctgtca aagtcatccc gcagagccgc gtcgccaagc cgcatcagcg cgagaagatc 240
ctaaatgaga ttgagctgca ccgagacctg cagcaccgcc acatcgtgcg tttttcgcac 300
cactttgagg acgctgacaa catctacatt ttcttggagc tctgcagccg aaagtccctg 360
gcccacatct ggaaggcccg gcacaccctg ttggagccag aagtgcgcta ctacctgcgg 420
cagatccttt ctggcctcaa gtacttgcac cagcgcggca tcttgcaccg ggacctcaag 480
ttgggaaatt ttttcatcac tgagaacatg gaactgaagg tgggggattt tgggctggca 540
gcccggttgg agcctccgga gcagaggaag aagaccatct gtggcacccc caactatgtg 600
gctccagaag tgctgctgag acagggccac ggccctgaag cggatgtatg gtcactgggc 660

tgtgtcatgt acacgctgct ctgcgggagc cctccctttg agacggctga cctgaaggag 720
acgtaccgct gcatcaagca ggttcactac acgctgcctg ccagcctctc actgcctgcc 780
cggcagctcc tggccgccat ccttcgggcc tcaccccgag accgcccctc tattgaccag 840
atcctgcgcc atgacttctt taccaagggc tacacccccg atcgactccc tatcagcagc 900
tgcgtgacag tcccagacct gacacccccc aacccagcta ggagtctgtt tgccaaagtt 960
accaagagcc tctttggcag aaagaagaag agtaagaatc atgcccagga gagggatgag 1020
gtctccggtt tggtgagcgg cctcatgcgc acatccgttg gccatcagga tgccaggcca 1080
gaggctccag cagcttctgg cccagcccct gtcagcctgg tagagacagc acctgaagac 1140
agctcacccc gtgggacact ggcaagcagt ggagatggat ttgaagaagg tctgactgtg 1200
gccacagtag tggagtcagc cctttgtgct ctgagaaatt gtatagcttt catgccccca 1260
gcggaacaga acccggcccc cctggcccag ccagagcctc tggtgtgggt cagcaagtgg 1320
gttgactact ccaataagtt cggctttggg tatcaactgt ccagccgccg tgtggctgtg 1380
ctcttcaacg atggcacaca tatggccctg tcggccaaca gaaagactgt gcactacaat 1440
cccaccagca caaagcactt ctccttctcc gtgggtgctg tgccccgggc cctgcagcct 1500
cagctgggta tcctgcggta cttcgcctcc tacatggagc agcacctcat gaagggtgga 1560
gatctgccca gtgtggaaga ggtagaggta cctgctccgc ccttgctgct gcagtgggtc 1620
aagacggatc aggctctcct catgctgttt agtgatggca ctgtccaggt gaacttctac 1680
ggggaccaca ccaagctgat tctcagtggc tgggagcccc tccttgtgac ttttgtggcc 1740
cgaaatcgta gtgcttgtac ttacctcgct tcccaccttc ggcagctggg ctgctctcca 1800
gacctgcggc agcgactccg ctatgctctg cgcctgctcc gggaccgcag cccagcttag 1860
gacccaagcc ctgaaggcct gaggcctgtg cctgtcaggc tctggccctt gcctttgtgg 1920
ccttccccct tcctttggtg cctcactggg ggctttgggc cgaatccccc agggaatcag 1980
ggaccagctt tactggagtt gggggcggct tgtcttcgct ggctcctacc ccatctccaa 2040
gataagcctg agccttagct cccagctagg gggcgttatt tatggaccac ttttatttat 2100
tgtcagacac ttatttattg ggatgtgagc cccagggggc ctcctcctag gataataaac 2160
aattttgca 2169

<210> 16
<211> 607
<212> PRT
<213> Homo sapiens

<220>
<223> Description of Sequence: N/A

<400> 16
Met Leu Ala Gly Leu Pro Thr Ser Asp Pro Gly Arg Leu Ile Thr Asp

1               5                   10                  15
Pro Arg Ser Gly Arg Thr Tyr Leu Lys Gly Arg Leu Leu Gly Lys Gly
            20                  25                  30
Gly Phe Ala Arg Cys Tyr Glu Ala Thr Asp Thr Glu Thr Gly Ser Ala
        35                  40                  45
Tyr Ala Val Lys Val Ile Pro Gln Ser Arg Val Ala Lys Pro His Gln
    50                  55                  60
Arg Glu Lys Ile Leu Asn Glu Ile Glu Leu His Arg Asp Leu Gln His
65                  70                  75                  80
Arg His Ile Val Arg Phe Ser His His Phe Glu Asp Ala Asp Asn Ile
                85                  90                  95
Tyr Ile Phe Leu Glu Leu Cys Ser Arg Lys Ser Leu Ala His Ile Trp
            100                 105                 110
Lys Ala Arg His Thr Leu Leu Glu Pro Glu Val Arg Tyr Tyr Leu Arg
        115                 120                 125
Gln Ile Leu Ser Gly Leu Lys Tyr Leu His Gln Arg Gly Ile Leu His
    130                 135                 140
Arg Asp Leu Lys Leu Gly Asn Phe Phe Ile Thr Glu Asn Met Glu Leu
145                 150                 155                 160
Lys Val Gly Asp Phe Gly Leu Ala Ala Arg Leu Glu Pro Pro Glu Gln
                165                 170                 175
Arg Lys Lys Thr Ile Cys Gly Thr Pro Asn Tyr Val Ala Pro Glu Val
            180                 185                 190
Leu Leu Arg Gln Gly His Gly Pro Glu Ala Asp Val Trp Ser Leu Gly
        195                 200                 205
Cys Val Met Tyr Thr Leu Leu Cys Gly Ser Pro Pro Phe Glu Thr Ala
    210                 215                 220
Asp Leu Lys Glu Thr Tyr Arg Cys Ile Lys Gln Val His Tyr Thr Leu
225                 230                 235                 240
Pro Ala Ser Leu Ser Leu Pro Ala Arg Gln Leu Leu Ala Ala Ile Leu
                245                 250                 255
Arg Ala Ser Pro Arg Asp Arg Pro Ser Ile Asp Gln Ile Leu Arg His
            260                 265                 270
Asp Phe Phe Thr Lys Gly Tyr Thr Pro Asp Arg Leu Pro Ile Ser Ser
        275                 280                 285
Cys Val Thr Val Pro Asp Leu Thr Pro Pro Asn Pro Ala Arg Ser Leu
    290                 295                 300
Phe Ala Lys Val Thr Lys Ser Leu Phe Gly Arg Lys Lys Lys Ser Lys

305                 310                 315                 320
Asn His Ala Gln Glu Arg Asp Glu Val Ser Gly Leu Val Ser Gly Leu
                325                 330                 335
Met Arg Thr Ser Val Gly His Gln Asp Ala Arg Pro Glu Ala Pro Ala
            340                 345                 350
Ala Ser Gly Pro Ala Pro Val Ser Leu Val Glu Thr Ala Pro Glu Asp
        355                 360                 365
Ser Ser Pro Arg Gly Thr Leu Ala Ser Ser Gly Asp Gly Phe Glu Glu
    370                 375                 380
Gly Leu Thr Val Ala Thr Val Val Glu Ser Ala Leu Cys Ala Leu Arg
385                 390                 395                 400
Asn Cys Ile Ala Phe Met Pro Pro Ala Glu Gln Asn Pro Ala Pro Leu
                405                 410                 415
Ala Gln Pro Glu Pro Leu Val Trp Val Ser Lys Trp Val Asp Tyr Ser
            420                 425                 430
Asn Lys Phe Gly Phe Gly Tyr Gln Leu Ser Ser Arg Arg Val Ala Val
        435                 440                 445
Leu Phe Asn Asp Gly Thr His Met Ala Leu Ser Ala Asn Arg Lys Thr
    450                 455                 460
Val His Tyr Asn Pro Thr Ser Thr Lys His Phe Ser Phe Ser Val Gly
465                 470                 475                 480
Ala Val Pro Arg Ala Leu Gln Pro Gln Leu Gly Ile Leu Arg Tyr Phe
                485                 490                 495
Ala Ser Tyr Met Glu Gln His Leu Met Lys Gly Gly Asp Leu Pro Ser
            500                 505                 510
Val Glu Glu Val Glu Val Pro Ala Pro Pro Leu Leu Leu Gln Trp Val
        515                 520                 525
Lys Thr Asp Gln Ala Leu Leu Met Leu Phe Ser Asp Gly Thr Val Gln
    530                 535                 540
Val Asn Phe Tyr Gly Asp His Thr Lys Leu Ile Leu Ser Gly Trp Glu
545                 550                 555                 560
Pro Leu Leu Val Thr Phe Val Ala Arg Asn Arg Ser Ala Cys Thr Tyr
                565                 570                 575
Leu Ala Ser His Leu Arg Gln Leu Gly Cys Ser Pro Asp Leu Arg Gln
            580                 585                 590
Arg Leu Arg Tyr Ala Leu Arg Leu Leu Arg Asp Arg Ser Pro Ala
        595                 600                 605

<210> 17

<211> 3492

<212> DNA

<213> Homo sapiens

<220> PTP-SL

<223> Description of Sequence: N/A

<400> 17

cagctaagac ccggagaggt ggaatttcac tttgaaattc ccttgcctcg tgagggccgg 60

cgctgggcat gctcagtagc cgcggcgctg ctgctgggct gctgggctgg cgcggagtcc 120

accctgccgt ctccgccttg gcttctgggc gtccagaagg ccaggcattt gccgcctctg 180

agcgcttctg ttccccttac ccgcaacctc ctactgctct tcctctctcc ctctcttagg 240

gaggttgaag ctggtgctgg tttctgtcgg cgccacagac tgactgctct gcaaacccca 300

gccgaggacc tgaatcccgg agactagaag acccttggcg gtggctcttt ctaatagcac 360

tttacctgaa gtggggtcgt ggtggagttt ctcctccacc tctcaatgca aacactatgc 420

ggagagcagt ctgcttccct gcgctgtgcc tgctccttaa tcttcacgct gcagggtgct 480

tttcaggaaa caatgatcat tttttggcaa ttaatcagaa gaagagtggg aagccggtat 540

tcatttataa gcattcacaa gacattgaga agagcctgga tatagcccca caaaaaatct 600

acagacatag ctaccattcc tcttccgaag ctcaagtaag caaacgccac cagattgtca 660

attcagcatt tcctagaccc gcatatgacc cgtctctcaa tctgctggcc atggatggtc 720

aagatcttga agtggaaaat ctcccaatcc cagcagcaaa tgtaattgtg gtgacactgc 780

aaatggatgt aaacaagctg aacataacct tgcttcggat cttccgccaa ggagtggctg 840

cagctttagg actcttaccc cagcaagtgc acatcaatcg cctcattgga aagaagaaca 900

gtattgaact gtttgtgtct cccataaacc gaaaaacagg aatttctgat gctctgccct 960

ctgaggaagt tcttcgttca cttaatatca atgttttgca tcaaagttta tcccagtttg 1020

gaattacaga agtctctcct gagaaaaatg ttttacaagg gcagcatgaa gcggacaaaa 1080

tctggagcaa agaaggattt tatgctgttg tcatttttct cagcatcttt gttattatag 1140

taacgtgttt gatgattctt tacagattaa aagaaagatt tcagctttcc ttaagacaag 1200

acaaagagaa aaaccaggag atccacctat cgcccatcac attacagcca gcactgtccg 1260

aggcaaagac agtccacagc atggtccaac ctgagcaggc cccaaaggta ctgaatgttg 1320

tcgtggaccc tcaaggccga ggtgctcctg agatcagagc taccaccgct acctctgttt 1380

gcccttctcc tttcaaaatg aagcccatag gacttcaaga gagaagaggg tccaacgtat 1440

ctcttacatt ggacatgagt agcttgggga acattgaacc ctttgtgtct ataccaacac 1500

cacgggagaa ggtagcaatg gagtatctgc agtcagccag ccgaattctc acaaggtctc 1560

agctgaggga cgtcgtggca agttcacatt tactccaaag tgaattcatg gaaataccga 1620

tgaactttgt ggatcccaaa gaaattgata ttccgcgtca tggaactaaa aatcgctata 1680
agaccatttt accaaatccc ctcagcagag tgtgtttaag accaaaaaat gtaaccgatt 1740
cattgagcac ctacattaat gctaattata ttaggggcta cagtggcaag gagaaagcct 1800
tcattgccac gcagggcccc atgatcaaca ccgtggatga tttctggcag atggtttggc 1860
aggaagacag ccctgtgatt gttatgatca caaaactcaa agaaaaaaat gagaaatgtg 1920
tgctatactg gccggaaaag agagggatat atggaaaagt tgaggttctg gttatcagtg 1980
taaatgaatg tgataactac accattcgaa accttgtctt aaagcaagga agccacaccc 2040
aacatgtgaa gcattactgg tacacctcat ggcctgatca caagactcca gacagtgccc 2100
agcccctcct acagctcatg ctggatgtag aagaagacag acttgcttcc cagggccgag 2160
ggcctgtggt tgtccactgc agtgcaggaa taggtagaac agggtgtttt attgctacat 2220
ccattggctg tcaacagctg aaagaagaag gagttgtgga tgcactaagc attgtctgcc 2280
agcttcgtat ggatagaggt ggaatggtcc aaaccagtga gcagtatgaa tttgtgcacc 2340
atgctctgtg cctgtatgag agcagacttt cagcagagac tgtccagtga gtcattgaag 2400
acttgtcaga ccatcaatct cttggggtga ttaatcaaat tacccaccca aggcttctag 2460
aaggagcttc ctgcaatgga aggaaggaga agctctgaag cccatgtatg gcatggattg 2520
tggaagactg ggcaacatat ttaagatttc cagctccttg tgtatatgaa tgcatttgta 2580
agcatccccc aaattattct gaaggttttt tgatgatgga ggtatgatag gtttatcaca 2640
cagcctaagg cagattttgt tttgtctgta ctgactctat ctgccacaca gaatgtatgt 2700
atgtaatatt cagtaataaa tgtcatcagg tgatgactgg atgagctgct gaagacattc 2760
gtattatgtg ttagatgctt taatgtttgc aaaatctgcc ttgtgaatgg actgtcagct 2820
gttaaactgt tcctgttttg aagtgctatt acctttctca gttaccagaa tcttgctgct 2880
aaagttgcaa gtgattgata atggattttt aacagagaag tctttgtttt tgaaaaacaa 2940
aaatcaaaaa cagtaactat tttatatgga aatgtgtctt gataatatta cctattaaat 3000
gtgtatttat agtccctcct atcaaacaat tacagagcac aatgattgtc attgggtata 3060
tatgtattta ctctctatta ttgggcataa aggtggcttc tgctccagaa ctctatccac 3120
tgtatttcca catcgtgagt cattttactt taaaagggaa aaacaaattt gtagcaactc 3180
tgaagtatca agagttttaa ctacttgact ctcttttgct aagaagggat ttttgaatat 3240
gctatctacc tggaatctct ctctcaacaa aaggtatatg ccttcaggaa tgatataatc 3300
tgtcccattt tcgaggctcc ttataaggac atttccatgt atgtccttac atttctgaaa 3360
gctttcaatc ttcaagagcc aaaaaaaatt aaaataacta ccctcagcaa acactagctg 3420
ttctgctcat atatgaattt ttaatgcagc aatgttgact ttgtttcata ctgccaataa 3480
actcttaata ct 3492

<210> 18
<211> 657
<212> PRT
<213> Homo sapiens

<220>

<223> Description of Sequence: N/A

<400> 18

Met Arg Arg Ala Val Cys Phe Pro Ala Leu Cys Leu Leu Leu Asn Leu
1               5                   10                  15

His Ala Ala Gly Cys Phe Ser Gly Asn Asn Asp His Phe Leu Ala Ile
            20                  25                  30

Asn Gln Lys Lys Ser Gly Lys Pro Val Phe Ile Tyr Lys His Ser Gln
        35                  40                  45

Asp Ile Glu Lys Ser Leu Asp Ile Ala Pro Gln Lys Ile Tyr Arg His
    50                  55                  60

Ser Tyr His Ser Ser Ser Glu Ala Gln Val Ser Lys Arg His Gln Ile
65                  70                  75                  80

Val Asn Ser Ala Phe Pro Arg Pro Ala Tyr Asp Pro Ser Leu Asn Leu
                85                  90                  95

Leu Ala Met Asp Gly Gln Asp Leu Glu Val Glu Asn Leu Pro Ile Pro
            100                 105                 110

Ala Ala Asn Val Ile Val Val Thr Leu Gln Met Asp Val Asn Lys Leu
        115                 120                 125

Asn Ile Thr Leu Leu Arg Ile Phe Arg Gln Gly Val Ala Ala Ala Leu
    130                 135                 140

Gly Leu Leu Pro Gln Gln Val His Ile Asn Arg Leu Ile Gly Lys Lys
145                 150                 155                 160

Asn Ser Ile Glu Leu Phe Val Ser Pro Ile Asn Arg Lys Thr Gly Ile
                165                 170                 175

Ser Asp Ala Leu Pro Ser Glu Glu Val Leu Arg Ser Leu Asn Ile Asn
            180                 185                 190

Val Leu His Gln Ser Leu Ser Gln Phe Gly Ile Thr Glu Val Ser Pro
        195                 200                 205

Glu Lys Asn Val Leu Gln Gly Gln His Glu Ala Asp Lys Ile Trp Ser
    210                 215                 220

Lys Glu Gly Phe Tyr Ala Val Val Ile Phe Leu Ser Ile Phe Val Ile
225                 230                 235                 240

Ile Val Thr Cys Leu Met Ile Leu Tyr Arg Leu Lys Glu Arg Phe Gln
                245                 250                 255

Leu Ser Leu Arg Gln Asp Lys Glu Lys Asn Gln Glu Ile His Leu Ser

            260                 265                 270
Pro Ile Thr Leu Gln Pro Ala Leu Ser Glu Ala Lys Thr Val His Ser
        275                 280                 285
Met Val Gln Pro Glu Gln Ala Pro Lys Val Leu Asn Val Val Val Asp
    290                 295                 300
Pro Gln Gly Arg Gly Ala Pro Glu Ile Arg Ala Thr Thr Ala Thr Ser
305                 310                 315                 320
Val Cys Pro Ser Pro Phe Lys Met Lys Pro Ile Gly Leu Gln Glu Arg
                325                 330                 335
Arg Gly Ser Asn Val Ser Leu Thr Leu Asp Met Ser Ser Leu Gly Asn
            340                 345                 350
Ile Glu Pro Phe Val Ser Ile Pro Thr Pro Arg Glu Lys Val Ala Met
        355                 360                 365
Glu Tyr Leu Gln Ser Ala Ser Arg Ile Leu Thr Arg Ser Gln Leu Arg
    370                 375                 380
Asp Val Val Ala Ser Ser His Leu Leu Gln Ser Glu Phe Met Glu Ile
385                 390                 395                 400
Pro Met Asn Phe Val Asp Pro Lys Glu Ile Asp Ile Pro Arg His Gly
                405                 410                 415
Thr Lys Asn Arg Tyr Lys Thr Ile Leu Pro Asn Pro Leu Ser Arg Val
            420                 425                 430
Cys Leu Arg Pro Lys Asn Val Thr Asp Ser Leu Ser Thr Tyr Ile Asn
        435                 440                 445
Ala Asn Tyr Ile Arg Gly Tyr Ser Gly Lys Glu Lys Ala Phe Ile Ala
    450                 455                 460
Thr Gln Gly Pro Met Ile Asn Thr Val Asp Asp Phe Trp Gln Met Val
465                 470                 475                 480
Trp Gln Glu Asp Ser Pro Val Ile Val Met Ile Thr Lys Leu Lys Glu
                485                 490                 495
Lys Asn Glu Lys Cys Val Leu Tyr Trp Pro Glu Lys Arg Gly Ile Tyr
            500                 505                 510
Gly Lys Val Glu Val Leu Val Ile Ser Val Asn Glu Cys Asp Asn Tyr
        515                 520                 525
Thr Ile Arg Asn Leu Val Leu Lys Gln Gly Ser His Thr Gln His Val
    530                 535                 540
Lys His Tyr Trp Tyr Thr Ser Trp Pro Asp His Lys Thr Pro Asp Ser
545                 550                 555                 560
Ala Gln Pro Leu Leu Gln Leu Met Leu Asp Val Glu Glu Asp Arg Leu

                565                 570                 575
Ala Ser Gln Gly Arg Gly Pro Val Val Val His Cys Ser Ala Gly Ile
            580                 585                 590
Gly Arg Thr Gly Cys Phe Ile Ala Thr Ser Ile Gly Cys Gln Gln Leu
        595                 600                 605
Lys Glu Glu Gly Val Val Asp Ala Leu Ser Ile Val Cys Gln Leu Arg
    610                 615                 620
Met Asp Arg Gly Gly Met Val Gln Thr Ser Glu Gln Tyr Glu Phe Val
625                 630                 635                 640
His His Ala Leu Cys Leu Tyr Glu Ser Arg Leu Ser Ala Glu Thr Val
                645                 650                 655
Gln

<210> 19
<211> 985
<212> DNA
<213> Homo sapiens

<220> HSP86
<223> Description of Sequence: N/A

<400> 19
ccggcccggt gtggctgtgc cgttggtcct gtgcggtcac ttagccaaga tgcctgagga 60
aacccagacc caagaccaac cgatggagga ggaggaggtt gagacgttcg cctttcaggc 120
agaaattgcc cagttgatgt cattgatcat caatactttc tactcgaaca aagagatctt 180
tctgagagag ctcatttcaa attcatcaga tgcattggac aaaatccggt atgaaagctt 240
gacagatccc agtaaattag actctgggaa agagctgcat attaacctta taccgaacaa 300
acaagatcga actctcacta ttgtggatac tggaattgga atgaccaagg ctgacttgat 360
caataacctt ggtactatcg ccaagtctgg gaccaaagcg ttcatggaag ctttgcaggc 420
tggtgcagat atctctatga ttggccagtt cggtgttggt ttttattctg cttatttggt 480
tgctgagaaa gtaactgtga tcaccaaaca taacgatgat gagcagtacg cttgggagtc 540
ctcagcaggg ggatcattca cagtgaggac agacacaggt gaacctatgg gtcgtggaac 600
aaaagttatc ctacacctga aagaagacca aactgagtac ttggaggaac gaagaataaa 660
ggagattgtg aagaaacatt ctcagtttat tggatatccc attactcttt ttgtggagaa 720
ggaacgtgat aaagaagtaa gcgatgatga ggctgaagaa aaggaagaca aagaagaaga 780
aaaagaaaaa gaagagaaag agtcggaaga caaacctgaa attgaagatg ttggttctga 840

tgaggaagaa gaaaagaagg atggtgacaa gaagaagaag aagaagatta aggaaaagta 900
catcgatcaa gaagagctca acaaaacaaa gcccatctgg accagaaatc ccgacgatat 960
tactaatgag gagtacggag aattc 985

<210> 20
<211> 312
<212> PRT
<213> Homo sapiens

<220>
<223> Description of Sequence: N/A

<400> 20
Met Pro Glu Glu Thr Gln Thr Gln Asp Gln Pro Met Glu Glu Glu Glu
1               5                   10                  15

Val Glu Thr Phe Ala Phe Gln Ala Glu Ile Ala Gln Leu Met Ser Leu
            20                  25                  30

Ile Ile Asn Thr Phe Tyr Ser Asn Lys Glu Ile Phe Leu Arg Glu Leu
        35                  40                  45

Ile Ser Asn Ser Ser Asp Ala Leu Asp Lys Ile Arg Tyr Glu Ser Leu
    50                  55                  60

Thr Asp Pro Ser Lys Leu Asp Ser Gly Lys Glu Leu His Ile Asn Leu
65                  70                  75                  80

Ile Pro Asn Lys Gln Asp Arg Thr Leu Thr Ile Val Asp Thr Gly Ile
                85                  90                  95

Gly Met Thr Lys Ala Asp Leu Ile Asn Asn Leu Gly Thr Ile Ala Lys
            100                 105                 110

Ser Gly Thr Lys Ala Phe Met Glu Ala Leu Gln Ala Gly Ala Asp Ile
        115                 120                 125

Ser Met Ile Gly Gln Phe Gly Val Gly Phe Tyr Ser Ala Tyr Leu Val
    130                 135                 140

Ala Glu Lys Val Thr Val Ile Thr Lys His Asn Asp Asp Glu Gln Tyr
145                 150                 155                 160

Ala Trp Glu Ser Ser Ala Gly Gly Ser Phe Thr Val Arg Thr Asp Thr
                165                 170                 175

Gly Glu Pro Met Gly Arg Gly Thr Lys Val Ile Leu His Leu Lys Glu
            180                 185                 190

Asp Gln Thr Glu Tyr Leu Glu Glu Arg Arg Ile Lys Glu Ile Val Lys
        195                 200                 205

Lys His Ser Gln Phe Ile Gly Tyr Pro Ile Thr Leu Phe Val Glu Lys
    210                 215                 220

Glu Arg Asp Lys Glu Val Ser Asp Asp Glu Ala Glu Glu Lys Glu Asp
225                 230                 235                 240

Lys Glu Glu Glu Lys Glu Lys Glu Glu Lys Glu Ser Glu Asp Lys Pro
                245                 250                 255

Glu Ile Glu Asp Val Gly Ser Asp Glu Glu Glu Glu Lys Lys Asp Gly
            260                 265                 270

Asp Lys Lys Lys Lys Lys Lys Ile Lys Glu Lys Tyr Ile Asp Gln Glu
        275                 280                 285

Glu Leu Asn Lys Thr Lys Pro Ile Trp Thr Arg Asn Pro Asp Asp Ile
    290                 295                 300

Thr Asn Glu Glu Tyr Gly Glu Phe
305                 310